# EUROPEAN PATENT APPLICATION

(11) **EP 2 141 243 A2**
(43) Date of publication of application: **06.01.2010**
(21) Application number: 09167669.2
(22) Date of filing: 16.10.2001
(51) Int. Cl.: C12P 21/06, C07K 14/47, C07K 14/525, C07K 16/00, C07K 16/24

(54) **Protein scaffolds for antibody mimics and other binding proteins**

(30) Priority: 16.10.2000 US 688566
(62) Divisional of application: 01981621.4
(71) Applicant: Bristol-Myers Squibb Company, Princeton NJ 08543-4000 (US)
(72) Inventor: Lipovsek, Dasa, Cambridge, MA 02138 (US); Wagner, Richard W., Cambridge, MA 02138 (US); Kuimelis, Robert G., Brighton, MA 02135 (US)
(74) Representative: Reitstötter - Kinzebach

(57) **Abstract**

Disclosed herein are proteins that include an immunoglobulin fold and that can be used as scaffolds. Also disclosed herein are nucleic acids encoding such proteins and the use of such proteins in diagnostic methods and in methods for evolving novel compound-binding species and their ligands.

## Description

### Background of the Invention

This invention relates to protein scaffolds useful, for example, for the generation of products having novel binding characteristics.

Proteins having relatively defined three-dimensional structures, commonly referred to as protein scaffolds; may be used as reagents for the design of engineered products. These scaffolds typically contain one or more regions which are amenable to specific or random sequence variation, and such sequence randomization is often carried out to produce libraries of proteins from which desired products may be selected. One particular area in which such scaffolds are useful is the field of antibody design.

A number of previous approaches to the manipulation of the mammalian immune system to obtain reagents or drugs have been attempted. These have included injecting animals with antigens of interest to obtain mixtures of polyclonal antibodies reactive against specific antigens, production of monoclonal antibodies in hybridoma cell culture (Koehler and Milstein, Nature 256:495, 1975), modification of existing monoclonal antibodies to obtain new or optimized recognition properties, creation of novel antibody fragments with desirable binding characteristics, and randomization of single chain antibodies (created by connecting the variable regions of the heavy and light chains of antibody molecules with a flexible peptide linker) followed by selection for antigen binding by phage display (Clackson et al., Nature 352:624, 1991).

In addition, several non-immunoglobulin protein scaffolds have been proposed for obtaining proteins with novel binding properties. For example, a "minibody" scaffold, which is related to the immunoglobulin fold, has been designed by deleting three beta strands from a heavy chain variable domain of a monoclonal antibody (Tramontano et al., J. Mol. Recognit. 7:9, 1994). This protein includes 61 residues and can be used to present two hypervariable loops. These two loops have been randomized and products selected for antigen binding, but thus far the framework appears to have somewhat limited utility due to solubility problems. Another framework used to display loops has been tendamistat, a 74 residue, six-strand beta sheet sandwich held together by two disulfide bonds (McConnell and Hoess, J. Mol. Biol. 250:460, 1995). This scaffold includes three loops, but, to date, only two of these loops have been examined for randomization potential.

Other proteins have been tested as frameworks and have been used to display randomized residues on alpha helical surfaces (Nord et al., Nat. Biotechnol. 15:772, 1997; Nord et al., Protein Eng. 8:601, 1995), loops between alpha helices in alpha helix bundles (Ku and Schultz, Proc. Natl. Acad. Sci. USA 92:6552, 1995), and loops constrained by disulfide bridges, such as those of the small protease inhibitors (Markland et al., Biochemistry 35:8045, 1996; Markland et al., Biochemistry 35:8058, 1996; Rottgen and Collins, Gene 164:243, 1995; Wang et al., J. Biol. Chem. 270:12250, 1995).

### Summary of the Invention

The present invention provides a new family of proteins capable of evolving to bind any compound of interest. These proteins, which generally make use of a scaffold derived from a fibronectin type III (Fn3) or Fn3-like domain, function in a manner characteristic of natural or engineered antibodies (that is, polyclonal, monoclonal, or single-chain antibodies) and, in addition, possess structural advantages. Specifically, the structure of these antibody mimics has been designed for optimal folding, stability, and solubility, even under conditions that normally lead to the loss of structure and function in antibodies.

These antibody mimics may be utilized for the purpose of designing proteins which are capable of binding to virtually any compound (for example, any protein) of interest. For example, the ¹⁰Fn3-based molecules described herein may be used as scaffolds which are subjected to directed evolution to form a population with one or more randomized Fn3 loops that are analogous by position and structure to the complementarity-determining regions (CDRs) of an antibody variable region, and/or to randomize Fn3's other three solvent exposed loops. Such a directed evolution approach results in the production of antibody-like molecules with high affinities for antigens of interest. In addition, the scaffolds described herein may be used to display defined exposed loops (for example, loops previously randomized and selected on the basis of antigen binding) in order to direct the evolution of molecules that bind to such introduced loops. A selection of this type may be carried out to identify recognition molecules for any individual CDR-like loop or, alternatively, for the recognition of two or all three CDR-like loops combined into a non-linear epitope.

Accordingly, in a first aspect, the present invention features randomized or mutated scaffold proteins. In particular, the invention features a non-antibody protein including a domain having an immunoglobulin-like fold, the non-antibody protein deriving from a reference protein by having a mutated amino acid sequence, wherein the non-antibody protein binds with a Kd at least as tight as 1 µM to a compound that is not bound as tightly by the reference protein.

In addition, the invention features a non-antibody protein deriving from a scaffold protein including a domain having an immunoglobulin-like fold, wherein the amino acid sequence of the domain in the derived protein is more than 50% identical to the amino acid sequence of the domain in the scaffold protein.

In yet another embodiment, the invention features a protein that includes a fibronectin type III domain having at least one randomized loop, the protein being characterized by the ability of the Fn3 domain to bind to a compound that is not bound by the corresponding naturally-occurring Fn3 domain.

In various preferred embodiments, any of these proteins of the invention bind to their target compounds with a Kd at least as tight as 500 nM, preferably, with a Kd at least as tight as 100 nM or 10 nM, and, more preferably, with a Kd at least as tight as 1 nM, 500 pM, 100 pM, or even 20 pM. The protein preferably contains one, two, or three mutated loops and at least one of the loops, and preferably two or all three of the loops, contributes to the binding of the protein to the compound. Additionally, the reference protein preferably lacks disulfide bonds, and the derivative protein may have at least one disulfide bond.

With respect to certain embodiments, the domain having an immunoglobulin-like fold preferably has a molecular mass less than 10 kD or greater than 7.5 kD, and, more preferably, has a molecular mass between 7.5-10 kD. The proteins of the invention may be monomers under physiological conditions or may be multimers, for example, dimers. In other preferred embodiments, the reference protein used to derive a mutated protein of the invention is a naturally-occurring mammalian protein (for example, a human protein); and the domain having an immunoglobulin-like fold is mutated and includes up to 50%, and preferably up to 34%, mutated amino acids as compared to the immunoglobulin-like fold of the reference protein. In addition, the domain having the immunoglobulin-like fold preferably consists of approximately 50-150 amino acids, and more preferably consists of approximately 50 amino acids.

Derivative proteins of the invention may be derived from any appropriate reference protein including, but not limited to, the preferred proteins, fibronectin or a fibronectin dimer, tenascin, N-cadherin, E-cadherin, ICAM, titin, GCSF-receptor, cytokine receptor, glycosidase inhibitor, antibiotic chromoprotein, myelin membrane adhesion molecule P0, CD8, CD4, CD2, class I MHC, T-cell antigen receptor, CD1, C2 and I-set domains of VCAM-1, I-set immunoglobulin domain of myosin-binding protein C, I-set immunoglobulin domain of myosin-binding protein H, I-set immunoglobulin domain of telokin, NCAM, twitchin, neuroglian, growth hormone receptor, erythropoietin receptor, prolactin receptor, interferon-gamma receptor, β-galactosidase/glucuronidase, β-glucuronidase, transglutaminase, T-cell antigen receptor, superoxide dismutase, tissue factor domain, cytochrome F, green fluorescent protein, GroEL, and thaumatin.

In further preferred embodiments of Fn3 domain-containing proteins, the fibronectin type III domain is a mammalian (for example, a human) fibronectin type III domain; and the protein includes the tenth module of the fibronectin type III (¹⁰Fn3) domain. In such proteins, compound binding is preferably mediated by either one, two, or three ¹⁰Fn3 loops. In other preferred embodiments, the second (DE) loop of ¹⁰Fn3 may be extended in length relative to the naturally-occurring module, or the ¹⁰Fn3 may lack an integrin-binding motif. In these molecules, the integrin-binding motif may be replaced by an amino acid sequence in which a polar amino acid-neutral amino acid-acidic amino acid sequence (in the N-terminal to C-terminal direction) replaces the integrin-binding motif; alternatively, one preferred sequence is serine-glycine-glutamate. In another preferred embodiment, the fibronectin type III domain-containing proteins of the invention lack disulfide bonds.

Any of the proteins of the invention (for example, the fibronectin type III domain-containing proteins) may be formulated as part of a fusion protein. If the fusion protein is to be used for compound binding or compound binding selections, the fusion protein includes a heterologous protein that does not itself bind to the compound of interest. The heterologous protein may, for example, be an antibody or antibody domain (such as an immunoglobulin F_{c} domain), a complement protein, a toxin protein, or an albumin protein. In addition, any of the proteins of the invention (for example, the fibronectin type III domain proteins) may be covalently bound to a nucleic acid (for example, an RNA), and the nucleic acid may encode the protein. Moreover, the protein may be a multimer, or, particularly if it lacks an integrin-binding motif, it may be formulated in a physiologically-acceptable carrier.

The present invention also features proteins that include a fibronectin type III domain having at least one mutation in a β-sheet sequence. Again, these proteins are characterized by their ability to bind to compounds that are not bound or are not bound as tightly by the corresponding naturally-occurring fibronectin domain.

Any of the proteins of the invention may be immobilized on a solid support (for example, a bead or chip), and these proteins may be arranged in any configuration on the solid support, including an array.

In a related aspect, the invention further features nucleic acids encoding any of the proteins of the invention. In preferred embodiments, the nucleic acid is DNA or RNA.

In another related aspect, the invention also features a method for generating a protein which includes a fibronectin type III domain and which is pharmaceutically acceptable to a mammal, involving removing the integrin-binding domain of said fibronectin type III domain. This method may be applied to any of the fibronectin type III domain-containing proteins described above and is particularly useful for generating proteins for human therapeutic applications. The invention also features such fibronectin type III domain-containing proteins which lack integrin-binding domains.

In yet another related aspect, the invention features methods of obtaining derivative non-antibody proteins which bind to compounds of interest. One such method involves: (a) providing a non-antibody scaffold protein including an immunoglobulin-like fold, wherein the scaffold protein does not bind to the compound with a Kd as tight as 1 µM; (b) generating mutated derivatives of the non-antibody scaffold protein, thereby producing a library of mutated proteins; (c) contacting the library with the compound; (d) selecting from the library at least one derivative protein which binds to the compound with a Kd at least as tight as 1 µM; and (e) optionally repeating steps (b) - (d) substituting for the non-antibody scaffold protein in repeated step (b) the product from the previous step (d). This technique may also be carried out with any of the proteins of the invention (for example, any of the fibronectin type III domain-containing proteins).

In yet other related aspects, the invention features screening methods which may be used to obtain or evolve randomized or mutated proteins of the invention capable of binding to compounds of interest, or to obtain or evolve compounds (for example, proteins) capable of binding to a particular protein containing a randomized or mutated motif. In addition, the invention features screening procedures which combine these two methods, in any order, to obtain either compounds or proteins of interest.

In particular, a first screening method, useful for the isolation or identification of randomized or mutated proteins of interest, involves: (a) contacting a compound of interest with a candidate protein, the candidate protein being a derivative non-antibody protein including a domain having an immunoglobulin-like fold, the non-antibody protein deriving from a reference protein by having a mutated amino acid sequence wherein the non-antibody protein binds with a Kd at least as tight as 1 µM to a compound that is not bound as tightly by the reference protein, wherein the contacting is carried out under conditions that allow compound-protein complex formation; and (b) obtaining, from the complex, the derivative protein that binds to the compound. This general technique may also be carried out with a fibronectin type III domain protein having at least one randomized or mutated loop.

The second screening method is for isolating or identifying a compound which binds to a protein of the invention. This method begins with a non-antibody protein including a domain having an immunoglobulin-like fold and deriving from a reference protein by having a mutated amino acid sequence, wherein the non-antibody protein binds with a Kd at least as tight as 1 µM to a compound that is not bound as tightly by the reference protein. This derivative protein is then contacted with a candidate compound, wherein the contacting is carried out under conditions that allow compound-protein complex formation, and the compound which binds to the derivative protein is obtained from the complex. Again, this general technique may be carried out with any protein of the invention, for example, a protein with a mutated fibronectin type III domain.

In addition, the invention features diagnostic methods which employ the proteins of the invention (for example, fibronectin type III scaffold proteins and their derivatives). Such diagnostic methods may be carried out on a sample (for example, a biological sample) to detect one analyte or to simultaneously detect many different analytes in the sample. The method may employ any of the scaffold molecules described herein. Preferably, the method involves (a) contacting the sample with a protein of the invention that binds to the compound analyte, the contacting being carried out under conditions that allow compound-protein complex formation; and (b) detecting the complex, and therefore the compound in the sample. In addition, this method may be used to quantitate, as well as detect, compound levels in a sample.

In preferred embodiments of any of the selection or diagnostic methods described herein, the protein of the invention binds to its target compound with a Kd at least as tight as 1 µM or 500 nM, preferably, with a Kd at least as tight as 100 nM or 10 nM, and, more preferably, with a Kd at least as tight as 1 nM, 500 pM, 100 pM, or even 20 pM. The protein preferably contains one, two, or three mutated loops and at least one of the loops, and preferably two or all three of the loops contributes to the binding of the protein to the compound. Additionally, the reference protein preferably lacks disulfide bonds, and the derivative protein may have at least one disulfide bond.

With respect to certain embodiments of the methods, the domain having an immunoglobulin-like fold preferably has a molecular mass less than 10 kD or greater than 7.5 kD, and, more preferably, has a molecular mass between 7.5-10 kD. The proteins of the invention may be monomers under physiological conditions or may be multimers, for example, dimers. In other preferred embodiments, the reference protein used to derive a mutated protein of the invention is a naturally-occurring mammalian protein (for example, a human protein); and the domain having an immunoglobulin-like fold is mutated and includes up to 50%, and preferably up to 34%, mutated amino acids as compared to the immunoglobulin-like fold of the reference protein. In addition, the domain having an immunoglobulin-like fold preferably consists of approximately 50-150 amino acids, and more preferably consists of approximately 50 amino acids.

Derivative proteins used in the methods of the invention may be derived from any appropriate reference protein including, but not limited to, the preferred proteins, fibronectin or a fibronectin dimer, tenascin, N-cadherin, E-cadherin, ICAM, titin, GCSF-receptor, cytokine receptor, glycosidase inhibitor, antibiotic chromoprotein, myelin membrane adhesion molecule P0, CD8, CD4, CD2, class I MHC, T-cell antigen receptor, CD1, C2 and I-set domains of VCAM-1, I-set immunoglobulin domain of myosin-binding protein C, I-set immunoglobulin domain of myosin-binding protein H, I-set immunoglobulin domain of telokin, NCAM, twitchin, neuroglian, growth hormone receptor, erythropoietin receptor, prolactin receptor, interferon-gamma receptor, β-galactosidase/glucuronidase, β-glucuronidase, transglutaminase, T-cell antigen receptor, superoxide dismutase, tissue factor domain, cytochrome F, green fluorescent protein, GroEL, and thaumatin.

In addition, the steps of the selection methods described herein may be repeated with further mutation or randomization being carried out between cycles. For example, for the methods involving a protein having a mutated or randomized fibronectin type III domain, at least one loop of the fibronectin type III domain of the protein obtained in step (b) may be mutated and steps (a) and (b) repeated using the further randomized protein, or the compound obtained in step (b) may be modified and steps (a) and (b) repeated using the further modified compound. In these methods, the compound is preferably a protein, and the fibronectin type III domain is preferably a mammalian (for example, a human) fibronectin type III domain. In other preferred embodiments, the protein includes the tenth module of the fibronectin type III domain (¹⁰Fn3), and binding is mediated by one, two, or three ¹⁰Fn3 loops. In addition, the second (DE) loop of ¹⁰Fn3 may be extended in length relative to the naturally-occurring module, or ¹⁰Fn3 may lack an integrin-binding motif. Again, as described above, the integrin-binding motif may be replaced by an amino acid sequence in which a basic amino acid-neutral amino acid-acidic amino acid sequence (in the N-terminal to C-terminal direction) replaces the integrin-binding motif; alternatively, one preferred replacement sequence is serine-glycine-glutamate.

The selection and diagnostic methods described herein may be carried out using any of the proteins of the invention (for example, a fibronectin type III domain-containing protein). In addition, any of these proteins may be formulated as part of a fusion protein with a heterologous protein (for example, an antibody or antibody domain (including an immunoglobulin F_{c} domain) that does not itself bind the compound of interest, or a complement protein, toxin protein, or albumin protein). In addition, selections and diagnostic methods may be carried out using the proteins of the invention (for example, the fibronectin type III domain proteins) covalently bound to nucleic acids (for example, RNAs or any nucleic acid which encodes the protein). Moreover, the selections and diagnostic methods may be carried out using these proteins (for example, the fibronectin domain-containing proteins) as monomers or as multimers, such as dimers.

Preferably, the selections and diagnostic methods involve the immobilization of the binding target on a solid support. Preferred solid supports include columns (for example, affinity columns, such as agarose-based affinity columns), microchips, or beads. Alternatively, the proteins (for example, the Fn3 domain-containing proteins) may be immobilized and contacted with one or more potential binding targets.

For the diagnostic methods, the compound is often a protein, but may also be any other analyte in a sample. Detection may be accomplished by any standard technique including, without limitation, radiography, fluorescence detection, mass spectroscopy, or surface plasmon resonance.

In a final aspect, the invention features a non-antibody protein that binds tumor necrosis factor-α (TNF-α) with a Kd at least as tight as 1 µM, the protein having a sequence that is less than 20% identical to TNF-α receptor (for example, a naturally-occurring TNF-α receptor, such as a mammalian or human TNF-α receptor).

In preferred embodiments, this protein includes a mutated fibronectin type III domain and the protein is mutated in the DE, BC, and FG loops. Preferably, the mutated FG loop is the same length as the wild-type FG loop. In other preferred embodiments, the protein includes an immunoglobulin-like fold (preferably, having a molecular mass less than 10 kD, greater than 7.5 kD, or between 7.5-10 kD) that consists of approximately 50-150 amino acids, and preferably, approximately 50 amino acids.

The TNF-α binders according to the invention bind TNF-α with a Kd at least as tight as 1 µM, preferably, at least as tight as 500 nM, 100 nM, or 10 nM, more preferably, at least as tight as 1 nM or 500 pM, and, most preferably, at least as tight as 100 pM or even 20 pM. Preferably, these proteins contain one, two, or three mutated loops, and at least one, and preferably two or all three of the loops, contribute to the binding of the non-antibody protein to TNF-α. In other preferred embodiments, the non-antibody protein has at least one disulfide bond, and the non-antibody protein is a monomer or dimer under physiological conditions.

The TNF-α binders may be immobilized on a solid support (for example, a chip or bead), and may be part of an array. In addition, any of the TNF-α binders may be joined to a heterologous protein (for example, a heterologous protein that is an antibody or an antibody domain that does not bind TNF-α, an immunoglobulin F_{c} domain, a complement protein, or an albumin protein).

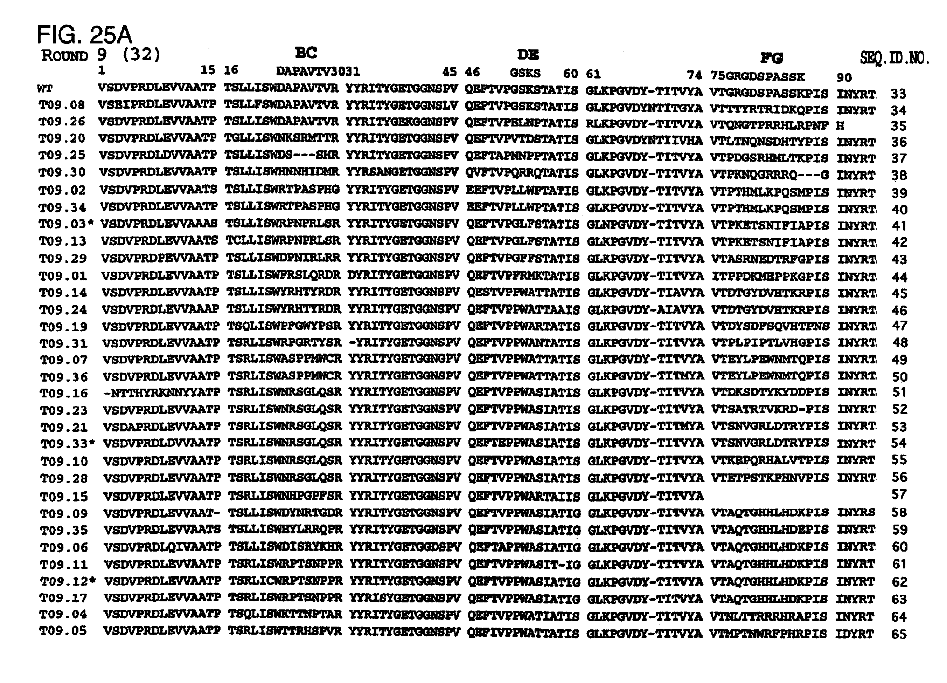

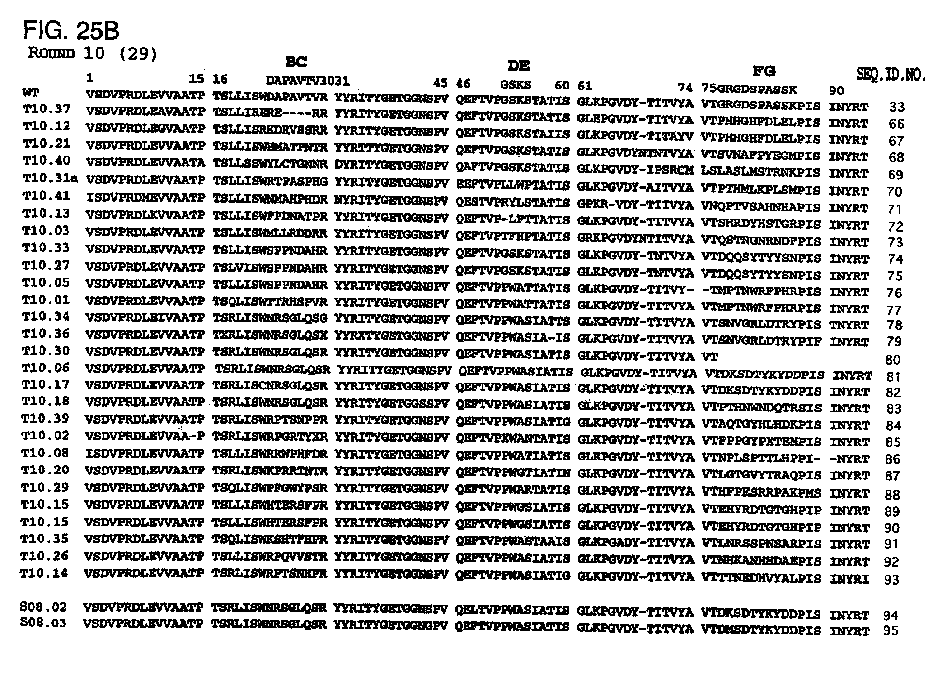

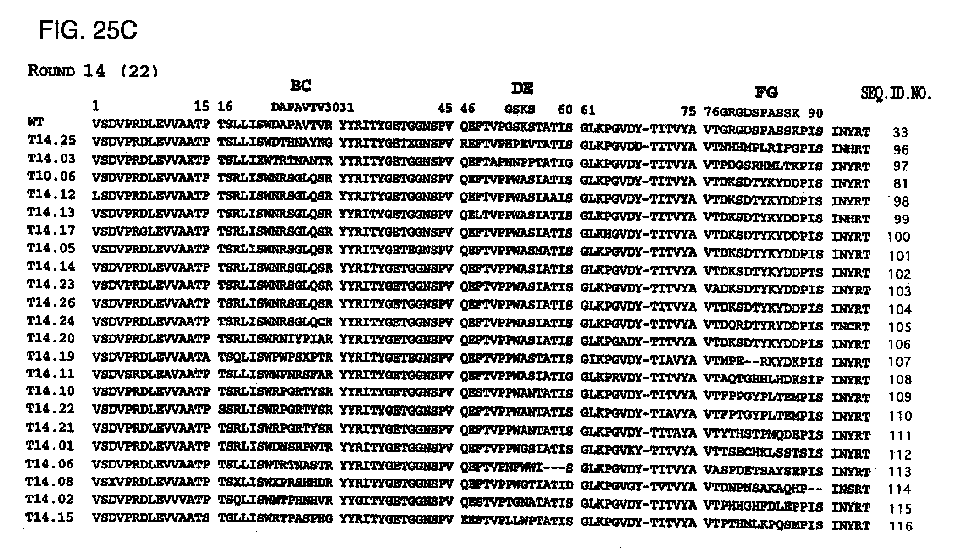

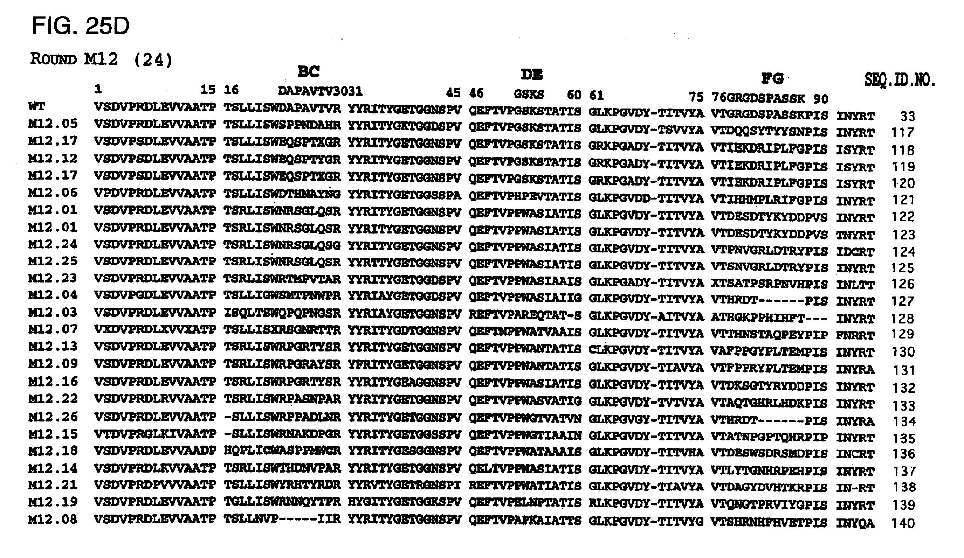

If desired, the protein may include a mutated fibronectin type III domain (for example, one derived from a human fibronectin type III domain, such as a mutated tenth module of the fibronectin type III domain (¹⁰Fn3)). In addition, the protein may lack an ¹⁰Fn3 integrin-binding motif. TNF-α binders preferably include a non-naturally occurring sequence in a loop of ¹⁰Fn3 (for example, the loop sequence PW(A/G), and may include a non-naturally occurring sequence in a β-sheer of ¹⁰Fn3. Particularly preferred TNF-α binders of the invention are shown in Figure 25 (SEQ ID NOS: 34-140).

In addition, in related aspects, the invention features nucleic acids encoding any of the TNF-α binding proteins of the invention, as well as a loop structure on any protein that includes any one of the amino acid sequences of Figure 25 (SEQ ID NOS: 34-140).

As used herein, by "non-antibody protein" is meant a protein that is not produced by the B cells of a mammal either naturally or following immunization of a mammal. This term also excludes antibody fragments of more than 100 amino acids, preferably, more than 80 amino acids, and, most preferably, more than 50 amino acids in length.

By "immunoglobulin-like fold" is meant a protein domain of between about 80-150 amino acid residues that includes two layers of antiparallel beta-sheets, and in which the flat, hydrophobic faces of the two beta-sheets are packed against each other. Proteins according to the invention may include several immunoglobulin-like folds covalently bound or associated non-covalently into larger structures.

By "scaffold" is meant a protein used to select or design a protein framework with specific and favorable properties, such as binding. When designing proteins from the scaffold, amino acid residues that are important for the framework's favorable properties are retained, while others residues may be varied. Such a scaffold has less than 50% of the amino acid residues that vary between protein derivatives having different properties and greater than or equal to 50% of the residues that are constant between such derivatives. Most commonly, these constant residues confer the same overall three-dimensional fold to all the variant domains, regardless of their properties.

By "fibronectin type III domain" is meant a domain having 7 or 8 beta strands which are distributed between two beta sheets, which themselves pack against each other to form the core of the protein, and further containing loops which connect the beta strands to each other and are solvent exposed. There are at least three such loops at each edge of the beta sheet sandwich, where the edge is the boundary of the protein perpendicular to the direction of the beta strands. Preferably, a fibronectin type III domain includes a sequence which exhibits at least 30% amino acid identity, and preferably at least 50% amino acid identity, to the sequence encoding the structure of the ¹⁰Fn3 domain referred to as "lttg" (ID = "lttg" (one ttg)) available from the RCSB (Research Collaboratory for Structural Bioinformatics) Protein Data Base. Sequence identity referred to in this definition is determined by the Homology program, available from Molecular Simulation (San Diego, CA). The invention further includes polymers of ¹⁰Fn3-related molecules, which are an extension of the use of the monomer structure, whether or not the subunits of the polyprotein are identical.

By "naturally occurring" is meant any protein that is encoded by a living organism.

By "randomized" or "mutated" is meant including one or more amino acid alterations relative to a template sequence. By "randomizing" or "mutating" is meant the process of introducing, into a sequence, such an amino acid alteration. Randomization or mutation may be accomplished through intentional, blind, or spontaneous sequence variation, generally of a nucleic acid coding sequence, and may occur by any technique, for example, PCR, error-prone PCR, or chemical DNA synthesis. By a "corresponding, non-mutated protein" is meant a protein that is identical in sequence, except for the introduced amino acid mutations.

By a "protein" is meant any sequence of two or more amino acids, regardless of length, post-translation modification, or function. "Protein" and "peptide" are used interchangeably herein.

By "RNA" is meant a sequence of two or more covalently bonded, naturally occurring or modified ribonucleotides. One example of a modified RNA included within this term is phosphorothioate RNA.

By "DNA" is meant a sequence of two or more covalently bonded, naturally occurring or modified deoxyribonucleotides.

By a "nucleic acid" is meant any two or more covalently bonded nucleotides or nucleotide analogs or derivatives. As used herein, this term includes, without limitation, DNA, RNA, and PNA.

By "pharmaceutically acceptable" is meant a compound or protein that may be administered to an animal (for example, a mammal) without significant adverse medical consequences.

By "physiologically acceptable carrier" is meant a carrier which does not have a significant detrimental impact on the treated host and which retains the therapeutic properties of the compound with which it is administered. One exemplary physiologically acceptable carrier is physiological saline. Other physiologically acceptable carriers and their formulations are known to one skilled in the art and are described, for example, in Remington's Pharmaceutical Sciences, (18th edition), ed. A. Gennaro, 1990, Mack Publishing Company, Easton, PA, incorporated herein by reference.

By a "fusion protein" is meant a protein that includes (i) a scaffold protein of the invention joined to (ii) a second, different (i.e., "heterologous") protein. "Fusion proteins" are distinguished from "nucleic acid-protein fusions" and "RNA-protein fusions" in that a "fusion protein" is composed entirely of amino acids, while both a "nucleic acid-protein fusion" and an "RNA-protein fusion" include a stretch of nucleic acids (the nucleic acid or RNA component) joined to a stretch of amino acids (the protein component).

By "selecting" is meant substantially partitioning a molecule from other molecules in a population. As used herein, a "selecting" step provides at least a 2-fold, preferably, at least a 30-fold, more preferably, at least a 100-fold, and, most preferably, at least a 1000-fold enrichment of a desired molecule relative to undesired molecules in a population following the selection step. A selection step may be repeated any number of times, and different types of selection steps may be combined in a given approach.

By "binding partner," as used herein, is meant any molecule which has a specific, covalent or non-covalent affinity for a portion of a desired compound (for example, protein) of interest. Examples of binding partners include, without limitation, members of antigen/antibody pairs, protein/inhibitor pairs, receptor/ligand pairs (for example cell surface receptor/ligand pairs, such as hormone receptor/peptide hormone pairs), enzyme/substrate pairs (for example, kinase/substrate pairs), lectin/carbohydrate pairs, oligomeric or heterooligomeric protein aggregates, DNA binding protein/DNA binding site pairs, RNA/protein pairs, and nucleic acid duplexes, heteroduplexes, or ligated strands, as well as any molecule which is capable of forming one or more covalent or non-covalent bonds (for example, disulfide bonds) with any portion of another molecule (for example, a compound or protein).

By a "solid support" is meant, without limitation, any column (or column material), bead, test tube, microtiter dish, solid particle (for example, agarose or sepharose), microchip (for example, silicon, silicon-glass, or gold chip), or membrane (for example, an inorganic membrane, nitrocellulose, or the membrane of a liposome or vesicle) to which an antibody mimic or an affinity complex may be bound, either directly or indirectly (for example, through other binding partner intermediates such as other antibodies or Protein A), or in which an antibody mimic or an affinity complex may be embedded (for example, through a receptor or channel).

The present invention provides a number of advantages. For example, as described in more detail below, the present antibody mimics exhibit improved biophysical properties, such as stability under reducing conditions and solubility at high concentrations. In addition, these molecules may be readily expressed and folded in prokaryotic systems, such as E. coli, in eukaryotic systems, such as yeast, and in in vitro translation systems, such as the rabbit reticulocyte lysate system. Moreover, these molecules are extremely amenable to affinity maturation techniques involving multiple cycles of selection, including in vitro selection using RNA-protein fusion technology (Roberts and Szostak, Proc. Natl. Acad. Sci USA 94:12297, 1997; Szostak et al., U.S.S.N. 09/007,005 and U.S.S.N. 09/247,190; Szostak et al. WO98/31700), phage display (see, for example, Smith and Petrenko, Chem. Rev. 97:317, 1997), and yeast display systems (see, for example, Boder and Wittrup, Nature Biotech. 15:553, 1997).

Other features and advantages of the present invention will be apparent from the following detailed description thereof, and from the claims.

### Brief Description of the Drawings

FIGURE 1 is a photograph showing a comparison between the structures of antibody heavy chain variable regions from camel (dark blue) and llama (light blue), in each of two orientations.
FIGURE 2 is a photograph showing a comparison between the structures of the camel antibody heavy chain variable region (dark blue), the llama antibody heavy chain variable region (light blue), and a fibronectin type III module number 10 (¹⁰Fn3) (yellow).
FIGURE 3 is a photograph showing a fibronectin type III module number 10 (¹⁰Fn3), with the loops corresponding to the antigen-binding loops in IgG heavy chains highlighted in red.
FIGURE 4 is a graph illustrating a sequence alignment between a fibronectin type III protein domain and related protein domains.
FIGURE 5 is a photograph showing the structural similarities between a ¹⁰Fn3 domain and 15 related proteins, including fibronectins, tenascins, collagens, and undulin. In this photograph, the regions are labeled as follows: constant, dark blue; conserved, light blue; neutral, white; variable, red; and RGD integrin-binding motif (variable), yellow.

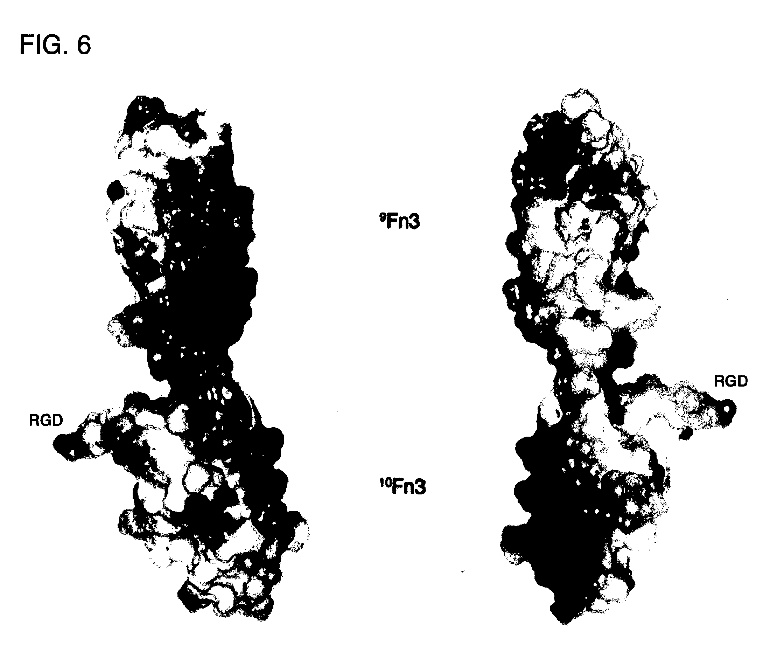

FIGURE 6 is a photograph showing space filling models of fibronectin III modules 9 and 10, in each of two different orientations. The two modules and the integrin binding loop (RGD) are labeled. In this figure, blue indicates positively charged residues, red indicates negatively charged residues, and white indicates uncharged residues.

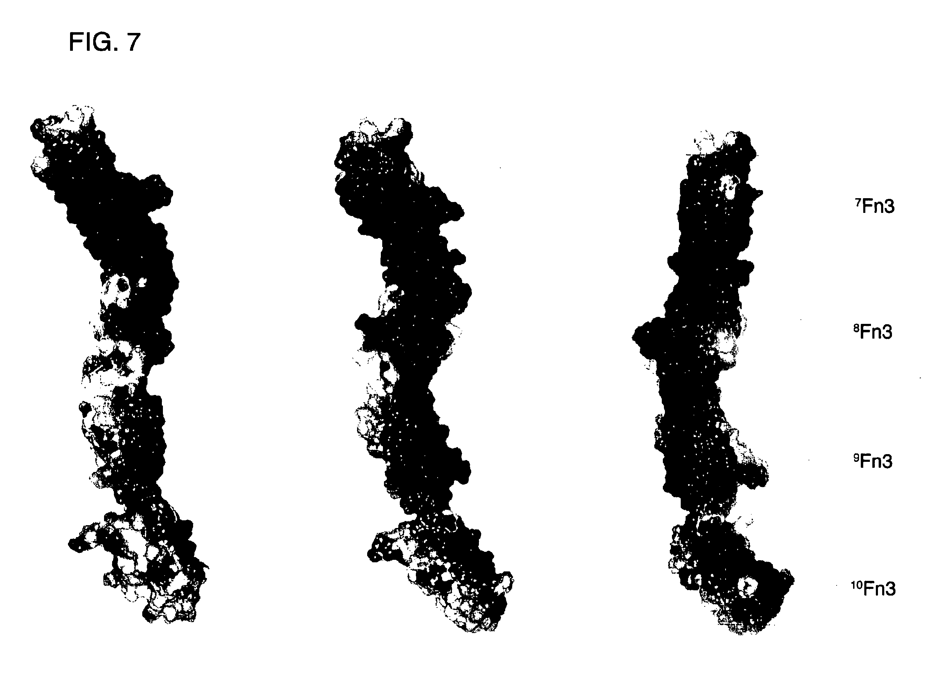

FIGURE 7 is a photograph showing space filling models of fibronectin III modules 7-10, in each of three different orientiations. The four modules are labeled. In this figure, blue indicates positively charged residues, red indicates negatively charged residues, and white indicates uncharged residues.
FIGURE 8 is a photograph illustrating the formation, under different salt conditions, of RNA-protein fusions which include fibronectin type III domains.
FIGURE 9 is a series of photographs illustrating the selection of fibronectin type III domain-containing RNA-protein fusions, as measured by PCR signal analysis.
FIGURE 10 is a graph illustrating an increase in the percent TNF-α binding during the selections described herein, as well as a comparison between RNA-protein fusion and free protein selections.
FIGURE 11 is a series of schematic representations showing IgG, ¹⁰Fn3, Fn-CH₁-CH₂-CH₃, and Fn-CH₂-CH₃ (clockwise from top left).
FIGURE 12 is a photograph showing a molecular model of Fn-CH₁-CH₂-CH₃ based on known three-dimensional structures of IgG (X-ray crystallography) and ¹⁰Fn3 (NMR and X-ray crystallography).
FIGURE 13 is a graph showing the time course of an exemplary ¹⁰Fn3-based nucleic acid-protein fusion selection of TNF-α binders. The proportion of nucleic acid-protein fusion pool (open diamonds) and free protein pool (open circles) that bound to TNF-α-Sepharose, and the proportion of free protein pool (full circles) that bound to underivatized Sepharose, are shown.
FIGURES 14 and 15 are graphs illustrating TNF-α binding by TNF-α Fn-binders. In particular, these figures show mass spectra data obtained from a ¹⁰Fn3 fusion chip and non-fusion chip, respectively.
FIGURES 16 and 17 are the phosphorimage and fluorescence scan, respectively, of an ¹⁰Fn3 array, illustrating TNF-α binding.
FIGURE 18 is a graph showing an alignment of the primary sequences of the llama V_{H} domain and the wild-type human ¹⁰Fn3 domain. Homologous residues between the two sequences are indicated. The ¹⁰Fn3 residues outside the randomized loops that were found to have mutated in approximately 45% of the selected clones are marked with arrows under the wild-type ¹⁰Fn3 sequence and with the letter that identifies the selected residue.
FIGURE 19 shows schematic representations of the llama V_{H} domain and the wild-type human ¹⁰Fn3 domain. The locations of the mutated framework residues are indicated.
FIGURE 20 is a graph illustrating the efficiency and specificity of binding of a free-protein pool translated from the original library (R0) and after ten rounds of selection with TNF-α (R10). Protein pool binding to underivatized Sepharose, to TNF-α-Sepharose, to IL-1α-Sepharose, and to IL-13-Sepharose is compared.
FIGURE 21 is a series of IgG-like scaffolds for the display of up to three loops.
FIGURE 22 is a series of IgG-like scaffolds for the display of up to four, or even six, loops.
FIGURE 23 is a series of scaffolds, unrelated to IgG, for the display of loop structures.
FIGURES 24A-24D are photographic and graphic illustrations demonstrating the specific capture of a target (TNF-α) by a mimic immobilized on a solid surface.

FIGURE 25 is a graph listing exemplary TNF-α binders (SEQ ID NOS: 33-140) according to the invention.

### Detailed Description

The novel antibody mimics described herein have been designed to be superior both to antibody-derived fragments and to non-antibody frameworks, for example, those frameworks cited above.

The major advantage of these antibody mimics over antibody fragments is structural. These antibody mimics are derived from whole, stable, and soluble structural scaffolds. For example, the Fn3 scaffold is found in the human body. Consequently, they exhibit better folding and thermostability properties than antibody fragments, whose creation involves the removal of parts of the antibody native fold, often exposing amino acid residues that, in an intact antibody, would be buried in a hydrophobic environment, such as an interface between variable and constant domains. Exposure of such hydrophobic residues to solvent increases the likelihood of aggregation of the antibody fragments.

In addition, the scaffolds described herein have no disulfide bonds, which have been reported to retard or prevent proper folding of antibody fragments under certain conditions. Since the present scaffolds do not rely on disulfides for native fold stability, they are stable under reducing conditions, unlike antibodies and their fragments which unravel upon disulfide bond reduction.

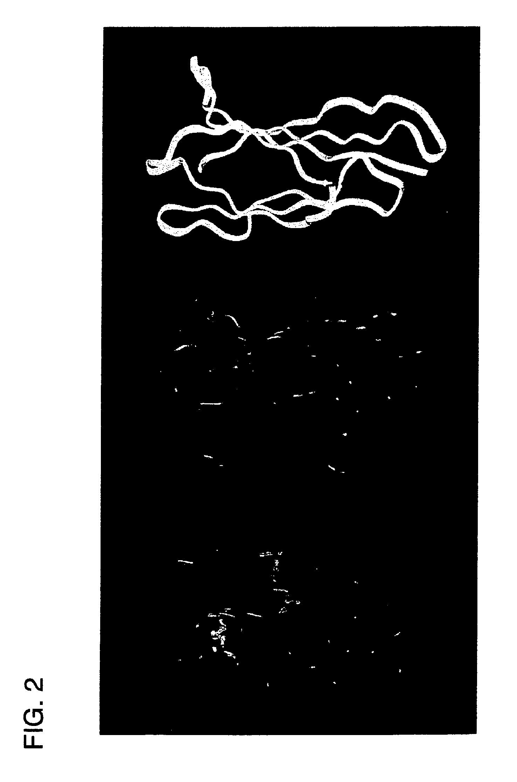

Moreover, these scaffolds provide the functional advantages of antibody molecules. In particular, despite the fact that the ¹⁰Fn3 module is not an immunoglobulin, its overall fold is close to that of the variable region of the IgG heavy chain (Figure 2), making it possible to display the three fibronectin loops analogous to CDRs in relative orientations similar to those of native antibodies. Because of this structure, the present antibody mimics possess antigen binding properties that are similar in nature and affinity to those of antibodies, and a loop randomization and shuffling strategy may be employed in vitro that is similar to the process of affinity maturation of antibodies in vivo.

There are now described below exemplary scaffolds, for example, fibronectin-based scaffolds, and their use for identifying, selecting, and evolving novel binding proteins as well as their target ligands. These examples are provided for the purpose of illustrating, and not limiting, the invention.

### ¹⁰Fn3 Structural Motif

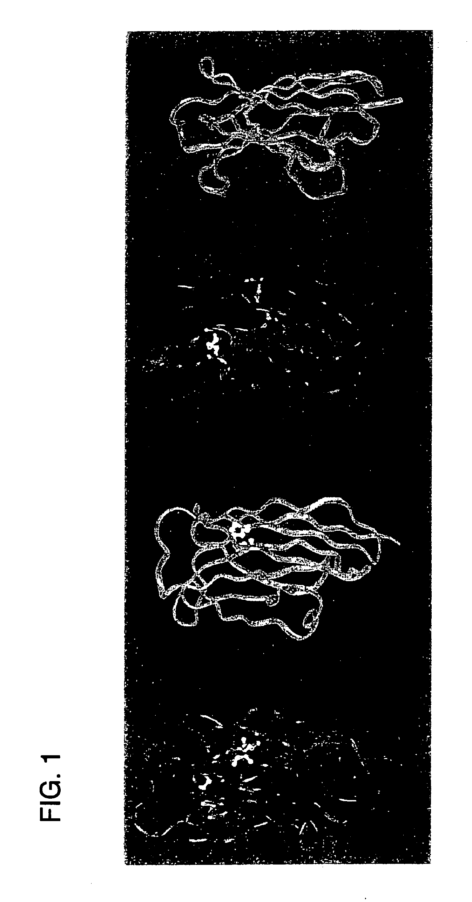

Preferred antibody mimics of the present invention are based on the structure of a fibronectin module of type III (Fn3), a common domain found in mammalian blood and structural proteins. This domain occurs more than 400 times in the protein sequence database and has been estimated to occur in 2% of the proteins sequenced to date, including fibronectins, tenascin, intracellular cytoskeletal proteins, and prokaryotic enzymes (Bork and Doolittle, Proc. Natl. Acad. Sci. USA 89:8990, 1992; Bork et al., Nature Biotech. 15:553, 1997; Meinke et al., J. Bacteriol. 175:1910, 1993; Watanabe et al., J. Biol. Chem. 265:15659, 1990). A particular scaffold is the tenth module of human Fn3 (¹⁰Fn3), which comprises 94 amino acid residues. The overall fold of this domain is closely related to that of the smallest functional antibody fragment, the variable region of the heavy chain, which comprises the entire antigen recognition unit in camel and llama IgG (Figure 1, 2). The major differences between camel and llama domains and the ¹⁰Fn3 domain are that (i) ¹⁰Fn3 has fewer beta strands (seven vs. nine) and (ii) the two beta sheets packed against each other are connected by a disulfide bridge in the camel and llama domains, but not in ¹⁰Fn3.

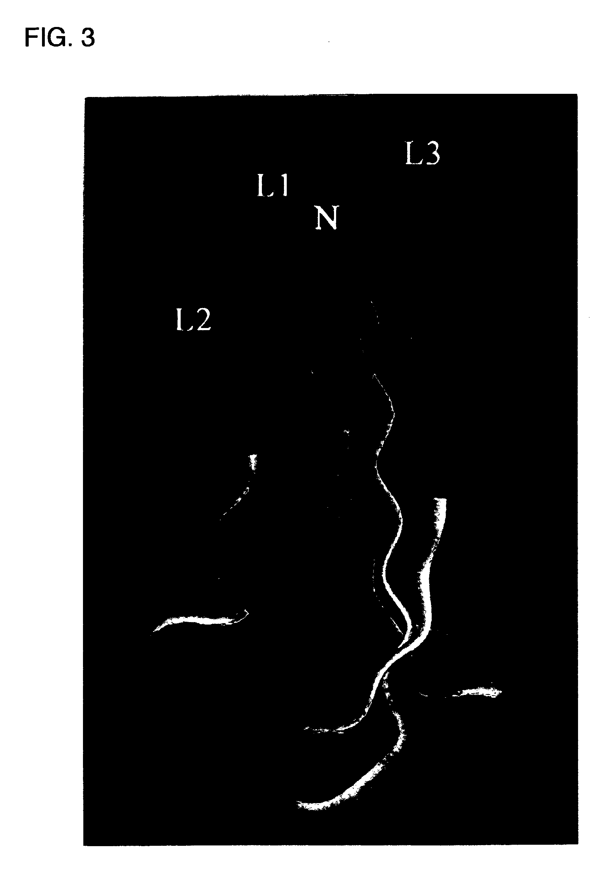

The three loops of ¹⁰Fn3 corresponding to the antigen-binding loops of the IgG heavy chain run between amino acid residues 21-31 (BC), 51-56 (DE), and 76-88 (FG) (Figure 3). The length of the BC and DE loop, 10 and 6 residues, respectively, fall within the narrow range of the corresponding antigen-recognition loops found in antibody heavy chains, that is, 7-10 and 4-8 residues, respectively. Accordingly, once randomized and selected for high antigen affinity, these two loops may make contacts with antigens equivalent to the contacts of the corresponding loops in antibodies.

In contrast, the FG loop of ¹⁰Fn3 is 12 residues long, whereas the corresponding loop in antibody heavy chains ranges from 4-28 residues. To optimize antigen binding, therefore, the length of the FG loop of ¹⁰Fn3 is preferably randomized in length as well as in sequence to cover the CDR3 range of 4-28 residues to obtain the greatest possible flexibility and affinity in antigen binding. Indeed, in general, the lengths as well as the sequences of the CDR-like loops of the antibody mimics may be randomized during in vitro or in vivo affinity maturation (as described in more detail below).

The tenth human fibronectin type III domain, ¹⁰Fn3, refolds rapidly even at low temperature; its backbone conformation has been recovered within I second at 5°C. Thermodynamic stability of ¹⁰Fn3 is high (ΔG_{U} = 24 kJ/mol = 5.7 kcal/mol), correlating with its high melting temperature of 110°C.

One of the physiological roles of ¹⁰Fn3 is as a subunit of fibronectin, a glycoprotein that exists in a soluble form in body fluids and in an insoluble form in the extracellular matrix (Dickinson et al., J. Mol. Biol. 236:1079, 1994). A fibronectin monomer of 220-250 kD contains 12 type I modules, two type II modules, and 17 fibronectin type III modules (Potts and Campbell, Curr. Opin.Cell Biol. 6:648, 1994). Different type III modules are involved in the binding of fibronectin to integrins, heparin, and chondroitin sulfate. ¹⁰Fn3 was found to mediate cell adhesion through an integrin-binding Arg-Gly-Asp (RGD) motif on one of its exposed loops. Similar RGD motifs have been shown to be involved in integrin binding by other proteins, such as fibrinogen, von Wellebrand factor, and vitronectin (Hynes et al., Cell 69:11, 1992). No other matrix- or cell-binding roles have been described for ¹⁰Fn3.

The observation that ¹⁰Fn3 has only slightly more adhesive activity than a short peptide containing RGD is consistent with the conclusion that the cell-binding activity of ¹⁰Fn3 is localized in the RGD peptide rather than distributed throughout the ¹⁰Fn3 structure (Baron et al., Biochemistry 31:2068, 1992). The fact that ¹⁰Fn3 without the RGD motif is unlikely to bind to other plasma proteins or extracellular matrix makes ¹⁰Fn3 a useful scaffold to replace antibodies. In addition, the presence of ¹⁰Fn3 in natural fibrinogen in the bloodstream suggests that ¹⁰Fn3 itself is unlikely to be immunogenic in the organism of origin.

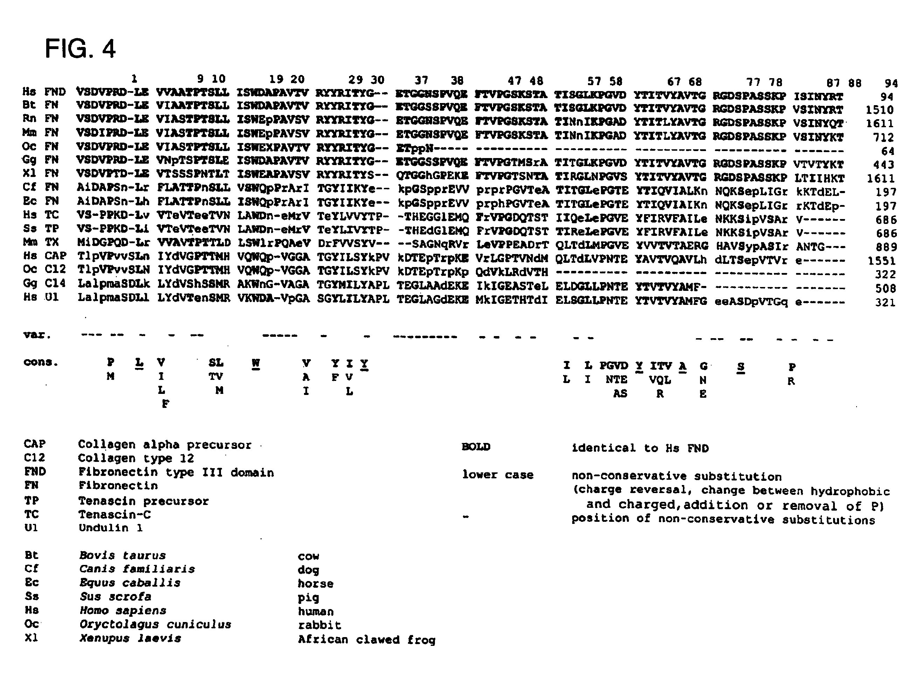

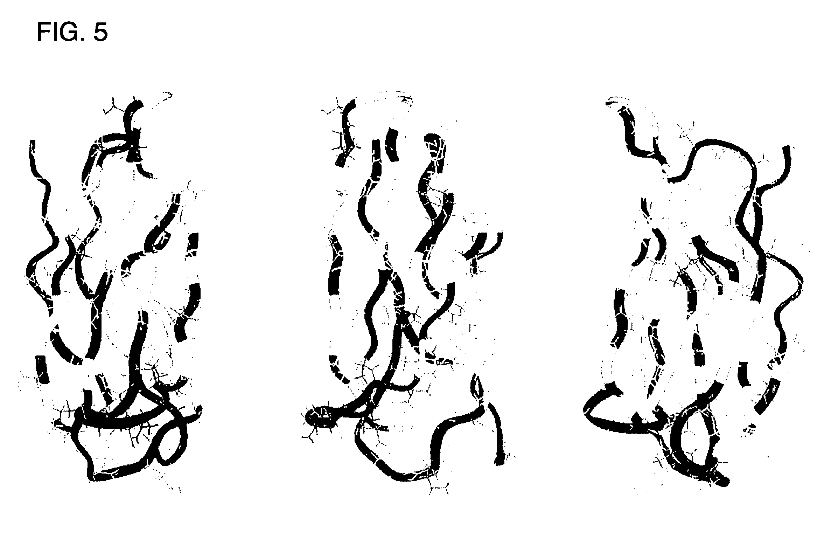

In addition, we have determined that the ¹⁰Fn3 framework possesses exposed loop sequences tolerant of randomization, facilitating the generation of diverse pools of antibody mimics. This determination was made by examining the flexibility of the ¹⁰Fn3 sequence. In particular, the human ¹⁰Fn3 sequence was aligned with the sequences of fibronectins from other sources as well as sequences of related proteins (Figure 4), and the results of this alignment were mapped onto the three-dimensional structure of the human ¹⁰Fn3 domain (Figure 5). This alignment revealed that the majority of conserved residues are found in the core of the beta sheet sandwich, whereas the highly variable residues are located along the edges of the beta sheets, including the N- and C-termini, on the solvent-accessible faces of both beta sheets, and on three solvent-accessible loops that serve as the hypervariable loops for affinity maturation of the antibody mimics. In view of these results, the randomization of these three loops are unlikely to have an adverse effect on the overall fold or stability of the ¹⁰Fn3 framework itself.

For the human ¹⁰Fn3 sequence, this analysis indicates that, at a minimum, amino acids 1-9, 44-50, 61-54, 82-94 (edges of beta sheets); 19, 21, 30-46 (even), 79-65 (odd) (solvent-accessible faces of both beta sheets); 21-31, 51-56, 76-88 (CDR-like solvent-accessible loops); and 14-16 and 36-45 (other solvent-accessible loops and beta turns) may be randomized to evolve new or improved compound-binding proteins. In addition, as discussed above, alterations in the lengths of one or more solvent exposed loops may also be included in such directed evolution methods.

Alternatively, changes in the β-sheet sequences may also be used to evolve new proteins. These mutations change the scaffold and thereby indirectly alter loop structure(s). If this approach is taken, mutations should not saturate the sequence, but rather few mutations should be introduced. Preferably, no more than between 3-20 changes should be introduced to the β-sheet sequences by this approach.

Sequence variation may be introduced by any technique including, for example, mutagenesis by Taq polymerase (Tindall and Kunkel, Biochemistry 27:6008 (1988)), fragment recombination, or a combination thereof. Similarly, an increase of the structural diversity of libraries, for example, by varying the length as well as the sequence of the CDR-like loops, or by structural redesign based on the advantageous framework mutations found in selected pools, may be used to introduce further improvements in antibody mimic scaffolds.

### Antibody Mimic Fusions

The antibody mimics described herein may be fused to other protein domains. For example, these mimics may be integrated with the human immune response by fusing the constant region of an IgG (F_{c}) with an antibody mimic, such as an ¹⁰Fn3 module, preferably through the C-terminus of ¹⁰Fn3. The F_{c} in such a ¹⁰Fn3-F_{c} fusion molecule activates the complement component of the immune response and increases the therapeutic value of the antibody mimic. Similarly, a fusion between an antibody mimic, such as ¹⁰Fn3, and a complement protein, such as C1q, may be used to target cells, and a fusion between an antibody mimic, such as ¹⁰Fn3, and a toxin may be used to specifically destroy cells that carry a particular antigen. In addition, an antibody scaffold, such as ¹⁰Fn3, in any form may be fused with albumin to increase its half-life in the bloodstream and its tissue penetration. Any of these fusions may be generated by standard techniques, for example, by expression of the fusion protein from a recombinant fusion gene constructed using publically available gene sequences.

### Scaffold Multimers

In addition to monomers, any of the scaffold constructs described herein may be generated as dimers or multimers of antibody mimics as a means to increase the valency and thus the avidity of antigen binding. Such multimers may be generated through covalent binding. For example, individual ¹⁰Fn3 modules may be bound by imitating the natural ⁸Fn3-⁹Fn3-¹⁰Fn3 C-to-N-terminus binding or by imitating antibody dimers that are held together through their constant regions. A ¹⁰Fn3-Fc construct may be exploited to design dimers of the general scheme of ¹⁰Fn3-Fc::Fc-¹⁰Fn3. The bonds engineered into the Fc::Fc interface may be covalent or non-covalent. In addition, dimerizing or multimerizing partners other than Fc can be used in hybrids, such as ¹⁰Fn3 hybrids, to create such higher order structures.

In particular examples, covalently bonded multimers may be generated by constructing fusion genes that encode the multimer or, alternatively, by engineering codons for cysteine residues into monomer sequences and allowing disulfide bond formation to occur between the expression products. Non-covalently bonded multimers may also be generated by a variety of techniques. These include the introduction, into monomer sequences, of codons corresponding to positively and/or negatively charged residues and allowing interactions between these residues in the expression products (and therefore between the monomers) to occur. This approach may be simplified by taking advantage of charged residues naturally present in a monomer subunit, for example, the negatively charged residues of fibronectin. Another means for generating non-covalently bonded antibody mimics is to introduce, into the monomer gene (for example, at the amino- or carboxy-termini), the coding sequences for proteins or protein domains known to interact. Such proteins or protein domains include coil-coil motifs, leucine zipper motifs, and any of the numerous protein subunits (or fragments thereof) known to direct formation of dimers or higher order multimers.

### Fibronectin-Like Molecules

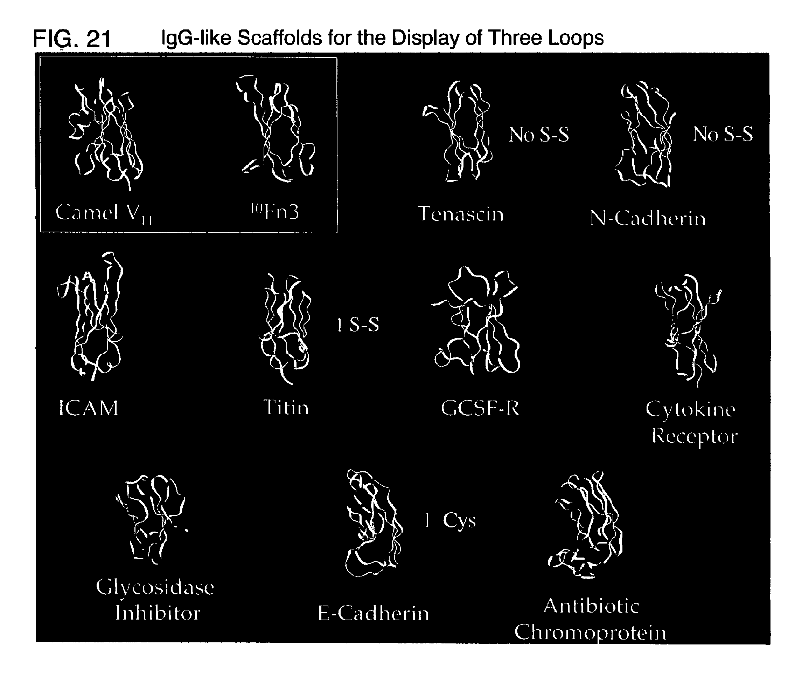

Although ¹⁰Fn3 represents a preferred scaffold for the generation of antibody mimics, other molecules may be substituted for ¹⁰Fn3 in the molecules described herein. These include, without limitation, human fibronectin modules ¹Fn3-⁹Fn3 and ¹¹Fn3-¹⁷Fn3 as well as related Fn3 modules from non-human animals and prokaryotes. In addition, Fn3 modules from other proteins with sequence homology to ¹⁰Fn3, such as tenascins and undulins, may also be used. Other exemplary scaffolds having immunoglobulin-like folds (but with sequences that are unrelated to the V_{H} domain) are shown in Figure 21 and include N-cadherin, ICAM-2, titin, GCSF receptor, cytokine receptor, glycosidase inhibitor, E-cadherin, and antibiotic chromoprotein. Yet further domains with related structures may be derived from myelin membrane adhesion molecule P0, CD8, CD4, CD2, class I MHC, T-cell antigen receptor, CD1, C2 and I-set domains of VCAM-1, I-set immunoglobulin domain of myosin-binding protein C, I-set immunoglobulin domain of myosin-binding protein H, I-set immunoglobulin domain of telokin, telikin, NCAM, twitchin, neuroglian, growth hormone receptor, erythropoietin receptor, prolactin receptor, GC-SF receptor, interferon-gamma receptor, β-galactosidase/glucuronidase, β-glucuronidase, and transglutaminase. Alternatively, any other protein that includes one or more immunoglobulin-like folds may be utilized. Such proteins may be identified, for example, using the program SCOP (Murzin et al., J. Mol. Biol. 247:536 (1995); Lo Conte et al., Nucleic Acids Res. 25:257 (2000).

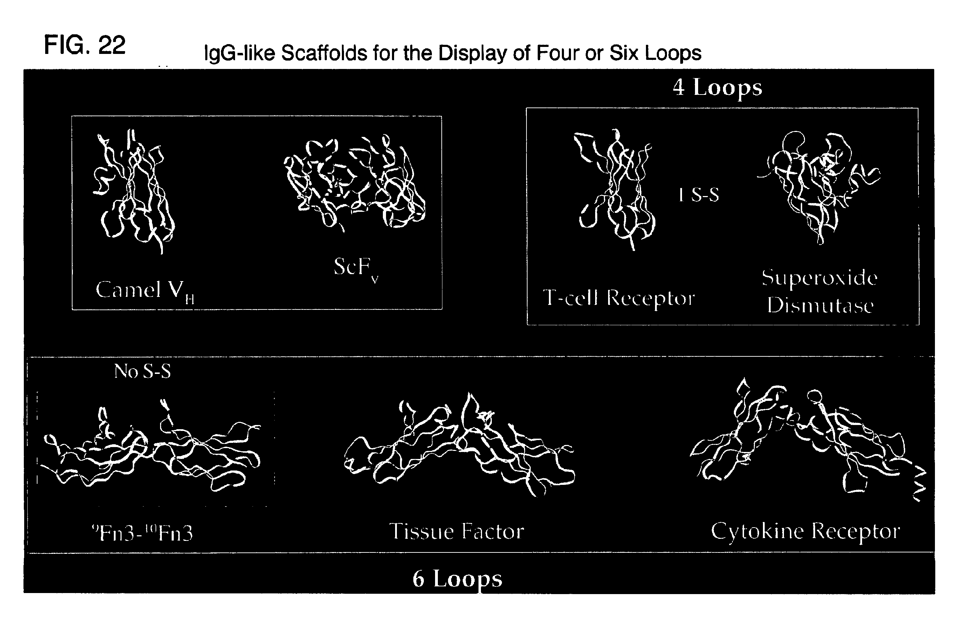

Generally, any molecule that exhibits a structural relatedness to the V_{H} domain (as identified, for example, using the computer program above) may be utilized as an antibody mimic. Such molecules may, like fibronectin, include three loops at the N-terminal pole of the molecule and three loops at the C-terminal pole, each of which may be randomized to create diverse libraries; alternatively, larger domains may be utilized, having larger numbers of loops, as long as a number of such surface randomizable loops are positioned closely enough in space that they can participate in antigen binding. Figure 22 shows examples of useful domains having more than three loops positioned close to each other. These examples include T-cell antigen receptor and superoxide dismutase, which each have four loops that can be randomized; and an Fn3 dimer, tissue factor domains, and cytokine receptor domains, each of which have three sets of two similar domains where three randomizable loops are part of the two domains (bringing the total number of loops to six).

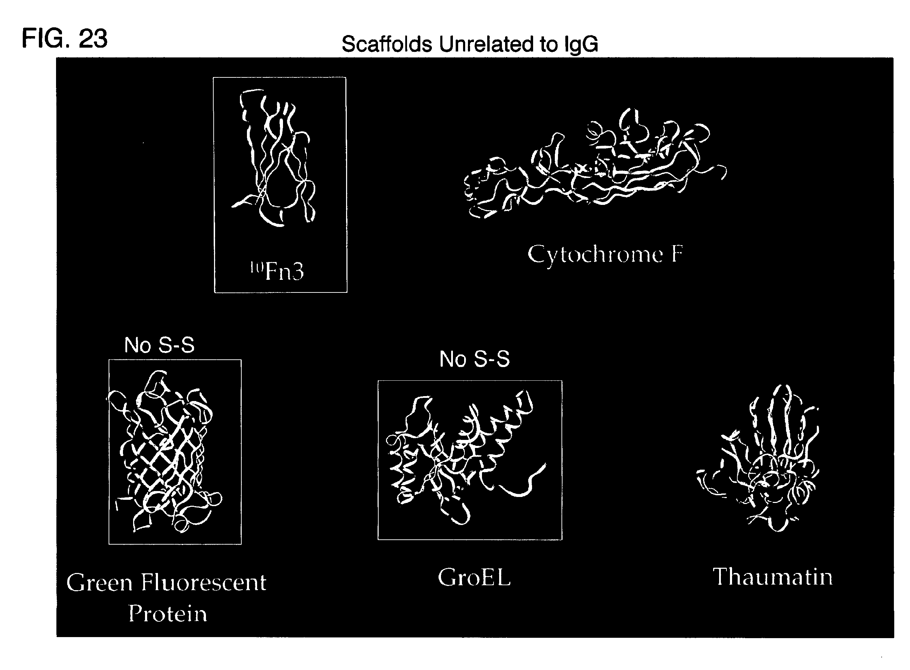

In yet another alternative, any protein having variable loops positioned close enough in space may be utilized for candidate binding protein production. For example, large proteins having spatially related, solvent accessible loops may be used, even if unrelated structurally to an immunoglobulin-like fold. Exemplary proteins include, without limitation, cytochrome F, green fluorescent protein, GroEL, and thaumatin (Figure 23). The loops displayed by these proteins may be randomized and superior binders selected from a randomized library as described herein. Because of their size, molecules may be obtained that exhibit an antigen binding surface considerably larger than that found in an antibody-antigen interaction. Other useful scaffolds of this type may also be identified using the program SCOP (Murzin et al., J. Mol. Biol. 247:536 (1995)) to browse among candidate proteins having numerous loops, particularly loops positioned among parallel beta sheets or a number of alpha-helices.

Modules from different organisms and parent proteins may be most appropriate for different applications. For example, in designing an antibody mimic, it may be most desirable to generate that protein from a fibronectin, or fibronectin-like molecule native to the organism for which a therapeutic is intended. In contrast, the organism of origin is less important or even irrelevant for antibody mimics that are to be used for in vitro applications, such as diagnostics, or as research reagents.

For any of these molecules, libraries may be generated and used to select binding proteins by any of the methods described herein.

### Directed Evolution of Scaffold-Based Binding Proteins

The antibody mimics described herein may be used in any technique for evolving new or improved binding proteins. In one particular example, the target of binding is immobilized on a solid support, such as a column resin or microtiter plate well, and the target contacted with a library of candidate scaffold-based binding proteins. Such a library may consist of antibody mimic clones, such as ¹⁰Fn3 clones constructed from the wild type ¹⁰Fn3 scaffold through randomization of the sequence and/or the length of the ¹⁰Fn3 CDR-like loops. If desired, this library may be an RNA-protein fusion library generated, for example, by the techniques described in Szostak et al., U.S.S.N. 09/007,005 and 09/247,190; Szostak et al., WO98/31700; and Roberts & Szostak, Proc. Natl. Acad. Sci. USA (1997) vol. 94, p. 12297-12302. Alternatively, it may be a DNA-protein library (for example, as described in Lohse, DNA-Protein Fusions and Uses Thereof, U.S.S.N. 60/110,549, U.S.S.N. 09/459,190, and WO 00/32823). The fusion library is incubated with the immobilized target, the support is washed to remove non-specific binders, and the tightest binders are eluted under very stringent conditions and subjected to PCR to recover the sequence information or to create a new library of binders which may be used to repeat the selection process, with or without further mutagenesis of the sequence. A number of rounds of selection may be performed until binders of sufficient affinity for the antigen are obtained.

In one particular example, the ¹⁰Fn3 scaffold may be used as the selection target. For example, if a protein is required that binds a specific peptide sequence presented in a ten residue loop, a single ¹⁰Fn3 clone is constructed in which one of its loops has been set to the length of ten and to the desired sequence. The new clone is expressed in vivo and purified, and then immobilized on a solid support. An RNA-protein fusion library based on an appropriate scaffold is then allowed to interact with the support, which is then washed, and desired molecules eluted and re-selected as described above.

Similarly, the scaffolds described herein, for example, the ¹⁰Fn3 scaffold, may be used to find natural proteins that interact with the peptide sequence displayed by the scaffold, for example, in an ¹⁰Fn3 loop. The scaffold protein, such as the ¹⁰Fn3 protein, is immobilized as described above, and an RNA-protein fusion library is screened for binders to the displayed loop. The binders are enriched through multiple rounds of selection and identified by DNA sequencing.

In addition, in the above approaches, although RNA-protein libraries represent exemplary libraries for directed evolution, any type of scaffold-based library may be used in the selection methods of the invention.

### Use

The antibody mimics described herein may be evolved to bind any antigen of interest. These proteins have thermodynamic properties superior to those of natural antibodies and can be evolved rapidly in vitro. Accordingly, these antibody mimics may be employed in place of antibodies in all areas in which antibodies are used, including in the research, therapeutic, and diagnostic fields. In addition, because these scaffolds possess solubility and stability properties superior to antibodies, the antibody mimics described herein may also be used under conditions which would destroy or inactivate antibody molecules. Finally, because the scaffolds of the present invention may be evolved to bind virtually any compound, these molecules provide completely novel binding proteins which also find use in the research, diagnostic, and therapeutic areas.

### Experimental Results

Exemplary scaffold molecules described above were generated and tested, for example, in selection protocols, as follows.

### Library construction

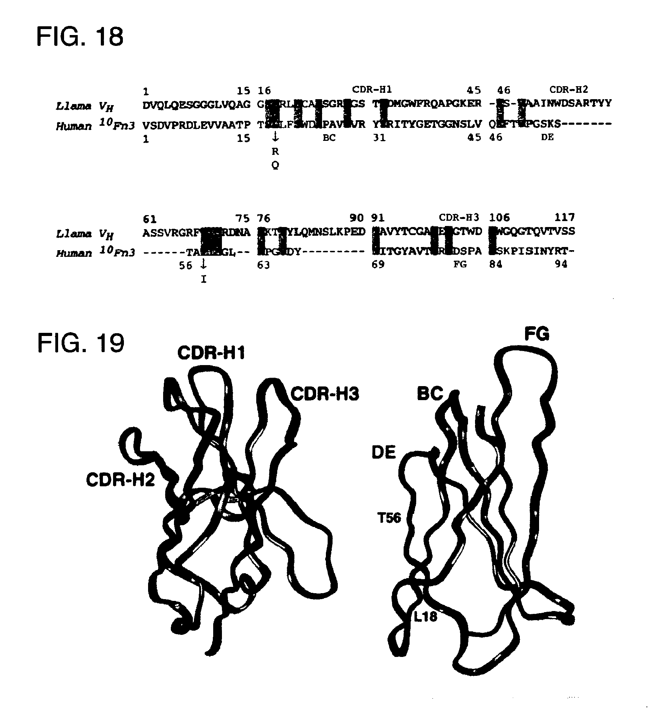

A complex library was constructed from three fragments, each of which contained one randomized area corresponding to a CDR-like loop. The randomized residues are indicated in Figure 18 as underlined sequences, specifically, residues 23-29 of the ¹⁰Fn3 BC loop (corresponding to CDR-H1 of the llama V_{H}); residues 52-55 of the ¹⁰Fn3 DE loop (corresponding to CDR-H2 of the llama V_{H}); and residues 78-87 of the ¹⁰Fn3 FG loop (corresponding to CDR-H3 of the llama V_{H}). The fragments were named BC, DE, and FG based on the names of the CDR-H-like loops contained within them; in addition to ¹⁰Fn3 and a randomized sequence, each of the fragments contained stretches encoding an N-terminal His₆ domain or a C-terminal FLAG peptide tag. At each junction between two fragments (i.e., between the BC and DE fragments or between the DE and FG fragments), each DNA fragment contained recognition sequences for the EarI Type IIS restriction endonuclease. This restriction enzyme allowed the splicing together of adjacent fragments while removing all foreign, non-¹⁰Fn3, sequences. It also allowed for a recombination-like mixing of the three ¹⁰Fn3 fragments between cycles of mutagenesis and selection.

The wild-type, human ¹⁰Fn3 gene was cloned from a human liver library (Maxim Biotech, South San Francisco, CA) using the primers Hu5PCR-NdeI 5'CATATGGTTTCTGATGTTCCGAGG3'; SEQ ID NO: 28) and Hu3PCR-EcoRI (5'GAATTCCTATGTTCGGTAATTAATGGAAATTG3'; SEQ ID NO: 29). Three different libraries were constructed from the wild-type segments obtained by the PCR of the ¹⁰Fn3 clone and from randomized segments obtained by oligonucleotide synthesis. The BCᵣ-DEᵣ-FGᵣ library was obtained by randomizing the selected residues in BC, DE, and FG loops; the BCᵣ-DE_{wt}-FGᵣ library was obtained by randomizing the selected residues in BC and FG loops, leaving the DE loop sequence wild-type; and the BC_{wt}-DE_{wt}-FGᵣ library was obtained by randomizing the selected residues in the FG loop only.

The BCᵣ, DEᵣ, and FGᵣ fragments were made synthetically. Each fragment was assembled from two overlapping oligonucleotides, which were first annealed, then extended to form the double-stranded DNA form of the fragment. The oligonucleotides that were used to construct and process the three fragments are listed below; the "Top" and "Bottom" species for each fragment are the oligonucleotides that contained the entire ¹⁰Fn3 encoding sequence. In these oligonucleotides designations, "N" indicates A, T, C, or G; and "S" indicates C or G.
HfnLbcTop (His): 
5'- GG AAT TCC TAA TAC GAC TCA CTA TAG GGA CAA TTA CTA TTT
ACA ATT ACA ATG CAT CAC CAT CAC CAT CAC GTT TCT GAT GTT
CCG AGG GAC CTG GAA GTT GTT GCT GCG ACC CCC ACC AGC-3'
(SEQ ID NO: 1)
HfnLbcTop (an alternative N-terminus): 
5'- GG AAT TCC TAA TAC GAC TCA CTA TAG GGA CAA TTA CTA TTT
ACA ATT ACA ATG GTT TCT GAT GTT CCG AGG GAC CTG GAA GTT
GTT GCT GCG ACC CCC ACC AGC-3' (SEQ ID NO: 2)
HFnLBCBot-flag8: 
5'-AGC GGA TGC CTT GTC GTC GTC GTC CTT GTA GTC GCT CTT CCC
TGT TTC TCC GTA AGT GAT CCT GTA ATA TCT (SNN)7 CCA GCT GAT
CAG TAG GCT GGT GGG GGT CGC AGC -3' (SEQ ID NO: 3)
HFnBC3'-flag8: 
5'-AGC GGA TGC CTT GTC GTC GTC GTC CTT GTA GTC GCT CTT CCC
TGT TTC TCC GTA AGT GAT CC-3' (SEQ ID NO: 4)
HFnLDETop: 
5'- GG AAT TCC TAA TAC GAC TCA CTA TAG GGA CAA TTA CTA TTT
ACA ATT ACA ATG CAT CAC CAT CAC CAT CAC CTC TTC ACA GGA
GGA AAT AGC CCT GTC C-3' (SEQ ID NO: 5)
HFnLDEBot-flag8: 
5'-AGC GGA TGC CTT GTC GTC GTC GTC CTT GTA GTC GCT CTT CGT
ATA ATC AAC TCC AGG TTT AAG GCC GCT GAT GGT AGC TGT
(SNN)4 AGG CAC AGT GAA CTC CTG GAC AGG GCT ATT TCC TCC
TGT -3' (SEQ ID NO: 6)
HFnDE3'-flag8: 
5'-AGC GGA TGC CTT GTC GTC GTC GTC CTT GTA GTC GCT CTT CGT
ATA ATC AAC TCC AGG TTT AAG G-3' (SEQ ID NO: 7)
HFnLFGTop: 
5'- GG AAT TCC TAA TAC GAC TCA CTA TAG GGA CAA TTA CTA TTT
ACA ATT ACA ATG CAT CAC CAT CAC CAT CAC CTC TTC TAT ACC
ATC ACT GTG TAT GCT GTC-3' (SEQ ID NO: 8)
HFnLFGBot-flag8: 
5'-AGC GGA TGC CTT GTC GTC GTC GTC CTT GTA GTC TGT TCG GTA
ATT AAT GGA AAT TGG (SNN)10 AGT GAC AGC ATA CAC AGT GAT
GGT ATA -3' (SEQ ID NO: 9)
HFnFG3'-flag8: 
5'-AGC GGA TGC CTT GTC GTC GTC GTC CTT GTA GTC TGT TCG GTA
ATT AAT GGA AAT TGG -3' (SEQ ID NO: 10)
T7Tmv (introduces T7 promoter and TMV untranslated region needed for in vitro translation): 
5'- GCG TAA TAC GAC TCA CTA TAG GGA CAA TTA CTA TTT ACA
ATT ACA-3' (SEQ ID NO: 11)
ASAflag8:
   5'-AGC GGA TGC CTT GTC GTC GTC GTC CTT GTA GTC-3' (SEQ ID NO: 12)
Unispl-s (spint oligonucleotide used to ligate mRNA to the puromycin-containing linker, described by Roberts et al, 1997, supra):
   5'-TTTTTTTTTNAGCGGATGC-3' (SEQ ID NO: 13)
A18---2PEG (DNA-puromycin linker):
   5'-(A)₁₈(PEG)₂CCPur (SEQ ID NO: 14)

The oligonucleotide pair BC_{Top} and BC_{Bot-flag8} was used to construct the fragment which contains the randomized BC loop; the pair DE_{Top} and DE_{Bot-flag8} was used to construct the fragment which contains the randomized DE loop; the pair BC_{Top} and DE_{3-Flag8} was used to PCR-amplify the BC_{wt} - DE_{wt} fragments; and the pair FG_{Top} and FG_{Bot-Flag8} was used to construct the fragment which contains the randomized FG loop. The pairs of oligonucleotides (500 pmol of each) were annealed in 100 µL of 10 mM Tris 7.5, 50 mM NaCl for 10 minutes at 85°C, followed by a slow (0.5-1 hour) cooling to room temperature. The annealed fragments with single-stranded overhangs were then extended using 100 U Klenow (New England Biolabs, Beverly, MA) for each 100 µL aliquot of annealed oligos, and the buffer made of 838.5 µl H₂O, 9 µl 1 M Tris 7.5, 5 µl 1M MgCl₂, 20 µl 10 mM dNTPs, and 7.5 µl 1M DTT. The extension reactions proceeded for 1 hour at 25°C.

In order to reduce the frequency of stop codons introduced by the random sequences, the randomized residues were encoded by (NNS)ₙ, where N stands for any nucleotide and S for an equimolar mixture of C and G; only one of the three stop codons (TAG) conforms to the NNS restriction. In addition to the sequence encoding ¹⁰Fn3, the gene fragments contained the 5' Tobacco Mosaic Virus (TMV) untranslated region and the T7 promoter, as well as the sequences encoding a 5' hexahistidine protein purification tag and a 3' FLAG epitope purification tag. In addition, as noted above, Ear I restriction endonuclease recognition sites were engineered into the overlaps between adjacent fragments in order to facilitate the assembly of the three fragments.

Next, each of the double-stranded fragments was transformed into an RNA-protein fusion (PROfusion™) using the technique developed by Szostak et al., U.S.S.N. 09/007,005 and U.S.S.N. 09/247,190; Szostak et al., WO98/31700; and Roberts & Szostak, Proc. Natl. Acad. Sci. USA (1997) vol. 94, p. 12297-12302. Briefly, the fragments were transcribed using an Ambion in vitro transcription kit, T7-MEGAshortscript™ (Ambion, Austin, TX), and the resulting mRNA was gel-purified and ligated to a 5'-phosphorylated DNA-puromycin linker, preferably, 5' dA₁₈PEG₂dCdCPur) using DNA ligase (Promega, Madison, WI); the mRNA was aligned with the DNA linker using a DNA splint oligonucleotide (5' TTTTTTTTTNAGCGGATGC 3'; SEQ ID NO: 30) as described in Szostak (supra). The mRNA-DNA-puromycin molecule was then translated using the Ambion rabbit reticulocyte lysate-based translation kit in the presence of ³⁵S-methionine. The resulting mRNA-DNA-puromycin-protein fusion was purified using Oligo(dT) cellulose, (Type 7, Amersham Pharmacia, Piscataway, NJ) and a complementary DNA strand was synthesized using reverse transcriptase (Superscript™II, Gibco, Life Technologies, Rockville, MD) and the RT primers described above (Unisplint-S or flagASA), following the manufacturer's instructions (preferably, a two-minute annealing at 70°C and a 40 minute reaction at 42°C).

The RNA-protein fusion with annealing cDNA obtained for each fragment was next purified on the resin appropriate to its peptide purification tag, i.e., on Ni-NTA agarose (Qiagen, Valencia, CA) for the His₆-tag and M2 Anti-Flag Agarose (Sigma, St. Louis, MO) for the FLAG-tag, following the procedures recommended by the manufacturers. The fragment-encoding genetic information recovered by KOH elution was amplified by PCR using Pharmacia Ready-to-Go PCR Beads, 10 pmol of 5' and 3' PCR primers, and the following PCR program (Pharmacia, Piscataway, NJ): Step 1: 95°C for 3 minutes; Step 2: 95°C for 30 seconds, 58/62°C for 30 seconds, 72°C for 1 minute, 20/25/30 cycles, as required; Step 3: 72°C for 5 minutes; Step 4: 4°C until end (typically, 25 cycles).

The resulting DNA was cleaved by 5-6 U EarI (New England Biolabs) per µg DNA; the reaction took place in T4 DNA Ligase Buffer (New England Biolabs) at 37°C, for 1 hour, and was followed by an optional incubation at 70°C for 15 minutes to inactivate Ear I. Equal amounts of the BC, DE, and FG fragments were combined and ligated to form a full-length ¹⁰Fn3 gene with randomized loops. The ligation required 10 U of fresh EarI (New England Biolabs) and 20 U of T4 DNA Ligase (Promega, Madison, WI), and took I hour at 37°C. Earl and ligase were then inactivated by a 15 minute incubation at 65°C.

Three different libraries, BC_{wt}-DE_{wt}-FGᵣ, BCᵣ-DE_{wt}-FGᵣ, and BCᵣ-DEᵣ-FGᵣ, were made in the manner described above. Each contained the form of the FG loop with 10 randomized residues. The BC and the DE loops of the first library bore the wild type ¹⁰Fn3 sequence; a BC loop with 7 randomized residues and a wild type DE loop made up the second library; and a BC loop with 7 randomized residues and a DE loop with 4 randomized residues made up the third library. The complexity of the FG loop in each of these three libraries was 10¹³; the further two randomized loops provided the potential for a complexity too large to be sampled in a laboratory. The combination of these libraries provided a master library having 10¹² unique clones.

The sequences of 76 randomly picked clones from the original, randomized, BCᵣ-DEᵣ-FGᵣ library showed no pattern in the randomized loops (data not shown); the amino acid frequency in the library varied in proportion to the number of codons available that encoded each residue, between 1% per position (glutamic acid, methionine, tryptophan) and 14% per position (proline). In contrast, the average probability for a residue in the preserved, beta-sheet framework to have remained as wild type was found to be 99%.

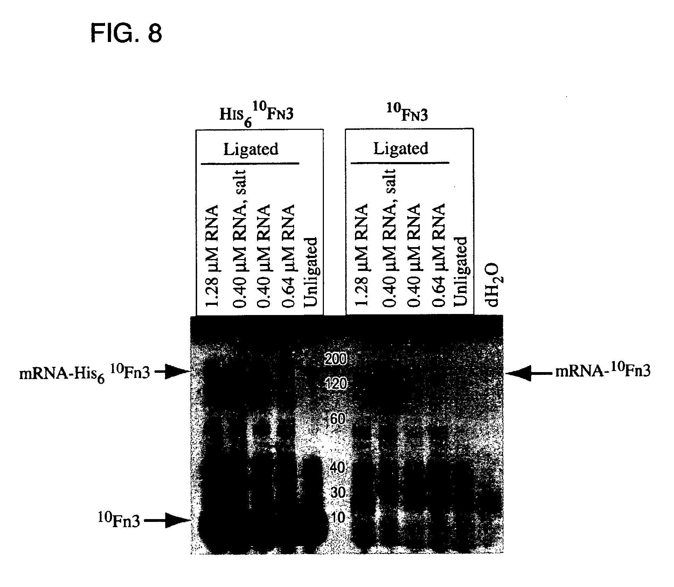

Equimolar amounts of the three libraries (2 pmoles of DNA each) were combined into one master library in order to simplify the selection process; target binding itself was expected to select the most suitable library for a particular challenge. RNA-protein fusions were obtained from the master library following the general procedure described in Szostak et al., U.S.S.N. 09/007,005 and 09/247,190; Szostak et al., WO98/31700; and Roberts & Szostak, Proc. Natl. Acad. Sci. USA (1997) vol. 94, p. 12297-12302 (Figure 8), except that affinity purification performed in rounds three to ten used only M2-Sepharose (see below).

### Fusion Selections

The master library in the RNA-protein fusion form was subjected to selection for binding to TNF-α (Pepro Tech, Rocky Hill, NJ). Two initial protocols were employed: one in which the target was immobilized on an agarose column and one in which the target was immobilized on a BIACORE chip. First, an extensive optimization of conditions to minimize background binders to the agarose column yielded the favorable buffer conditions of 50 mM HEPES pH 7.4, 0.02% Triton, 100 µg/ml sheared salmon sperm DNA. In this buffer, the non-specific binding of the ¹⁰Fn3-RNA fusion to TNF-α Sepharose was 0.3%. The non-specific binding background of the ¹⁰Fn3-RNA/cDNA library to TNF-α Sepharose was found to be 0.1%.

During each round of selection on TNF-α Sepharose, the library was first preincubated for an hour with underivatized Sepharose to remove any remaining non-specific binders; the flow-through from this pre-clearing was incubated for another hour with TNF-α Sepharose. The TNF-α Sepharose was washed for 3-30 minutes.

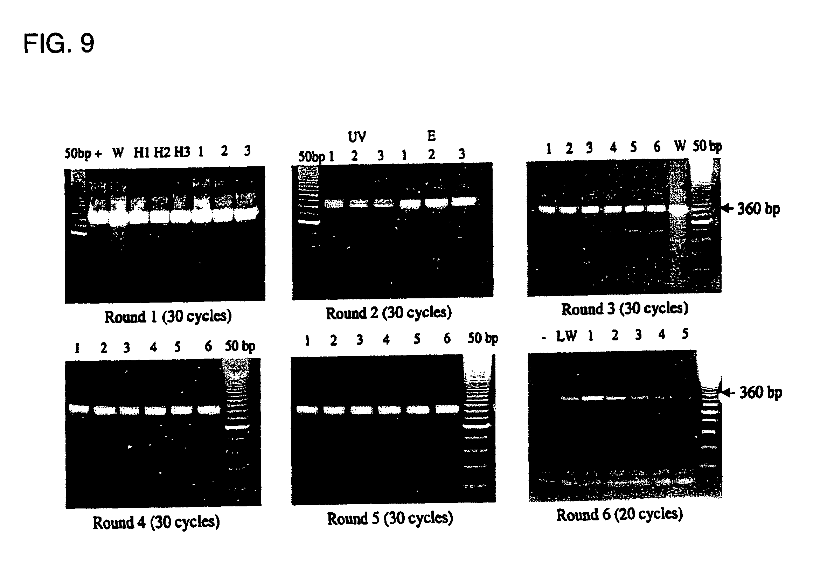

After each selection, the cDNA component of the complex that had been eluted from the solid support with 0.3 M NaOH or 0.1M KOH was amplified by PCR; a DNA band of the expected size persisted through multiple rounds of selection (Figure 9); similar results were observed in the two alternative selection protocols, and only the data from the agarose column selection is shown in Figure 9.

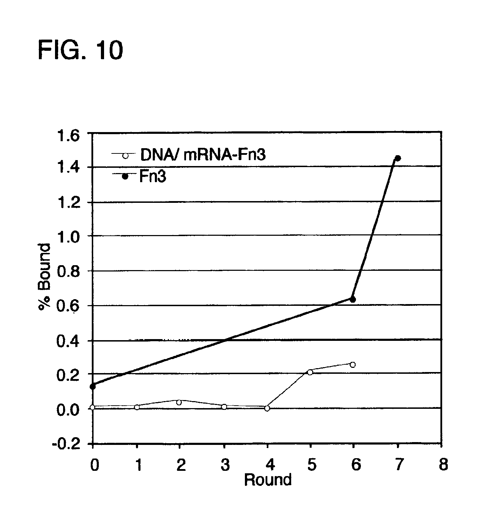

In this selection, in the first seven rounds, the binding of Fn3-RNA/cDNA molecules to the target remained low; in contrast, when free protein was translated from DNA pools at different stages of the selection, the proportion of the column binding species increased significantly between rounds (Figure 10).

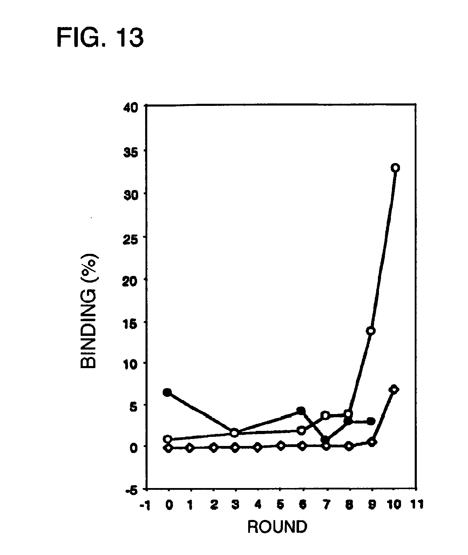

In later selections, the fusion pools selected in the first eight rounds of selection (R1-8) bound to TNF-α-Sepharose at levels close to the background (<0.25%) (Figure 13). After nine rounds of selection (R9), the binding of fusion to TNF-α-Sepharose increased sharply to 0.7%, and, after ten rounds of selection (R10), the binding increased further to 7% (Figure 13). These selections were carried out using TNF-α immobilized on Epoxy-Activated Sepharose™ 6B (Amersham Pharmacia) at 10 mg TNF/g Sepharose in 10 mL. Before use, the TNF-α-derivatized Sepharose was blocked in Binding Buffer (50 mM HEPES, pH 7.4, 0.02% Triton, 0.1 mg/mL sheared salmon sperm DNA (Ambion)), overnight, at 4°C.

The ¹⁰Fn3-based master library was transcribed, ligated to the puromycin-bearing linker, translated into an mRNA-protein library in the presence of 5-10 µL/300 µL ³⁵S-methionine, affinity purified on Oligo(dT) Cellulose, reverse-transcribed into a DNA/mRNA-protein library, and affinity-purified on M2-Sepharose (for rounds 3-10), as described above. Forty pmol of DNA/mRNA-protein fusion library molecules, the equivalent of 20 copies of 4 x 10¹² different sequences, were recovered, then subjected to the first round (R1) of the selection.

In the first step of the selection, 40 pmoles of the DNA/mRNA-protein library was incubated for 1 hour at 4°C, with tumbling, in 300 µL of Binding Buffer with 30 µL of Epoxy-Sepharose that had been subjected to the derivatization procedure in the absence of TNF-α. In the second round, 24 pmol of the library was added, and in the remaining eight rounds, 0.1-2 pmol of the library was added. The supernatant was recovered by microcentrifugation through a Micro Bio-Spin® chromatography column (BIO-RAD, Hercules, CA), then incubated with 30 µL of TNF-α-Sepharose (6 µM) in 300 µL of the Binding Buffer for 1 hour at 4°C (during Rounds 7-10, the Binding Buffer contained an additional 1 mg/mL ofBSA). The TNF-α-Sepharose was recovered on a spin column, then washed with 3 x 300 µL of Binding Buffer, eluted with 100 µL of 0.1 M KOH, and finally neutralized with 1 µL of 1 M Tris 8.0, 8 µL of 1 M HCI. Samples of the library, of the TNF-α-Sepharose before and after the elution, of the washes, and of the elutions were quantified by counting ³⁵S-methionine in the sample in a scintillation counter. The next round of selection began with the formation of a new DNA/mRNA-protein pool by PCR amplification, which was transcribed, translated, and reverse-transcribed from the PCR product.

The DNA pools obtained from the elution after nine and after ten rounds were cloned into the TOPO™ TA®, pCR2.1 cloning vector (Invitrogen, Carlsbad, CA) and transformed into *E*. *coli.* Between 30 and 100 clones were picked and grown into plasmid minipreps (Qiagen). Thirty-eight clones from R9 and 29 clones from R10 were picked at random and sequenced (DNA Sequencing Core Facility, Massachusetts General Hospital, Dept. of Molecular Biology, Boston, MA). The program ClustalW⁶⁰ was used to align the resulting protein sequences.

### Amino Acid Residue Sequences of the TNF-α Binding Clones

Thirty-eight of the 61 clones derived from R9 and from R10 had unique amino acid sequences, a surprising diversity. The ten clones that were isolated more than once, presumably because of their superior binding to TNF-α, are listed in Table 1 (full sequences in Figure 25).

Of the 61 clones picked randomly from the winning pool, only one (clone T09.08, sequence not shown) had its origin in the BC_{wt}-DE_{wt}-FGᵣ library, with another six from the BCᵣ-DE_{wt}-FGᵣ library. The observation that the remaining 54 (88% of the winners) were selected from the BCᵣ-DEᵣ-FGᵣ library points out the importance for TNF-α cooperative binding of the target by several loops.

The most common motif found in the selected loop sequences is PWA(S/T), which is found in the DE loop of 33 of the 61 clones; the more loosely defined sequence of PW(A/G) is seen in 41/61 clones. Such a strong selection for a specific DE sequence is surprising since the analogous CDR-H2 loops of antibody V_{H} domains generally make only a small contribution to antigen binding. On the other hand, the short length of the DE loop, which means that 10⁷ copies of each possible tetrapeptide sequence would be expected to be present in the library, would facilitate the optimization of any contribution of the DE loop to the selected properties. A survey of other Fn3 domains (Dickenson et al., J. Mol. Biol. 236:1079-1092 (1994)) shows that proline is found at positions equivalent to the ¹⁰Fn3 residue 52 as frequently as is the wild-type glycine; similarly, alanine, glycine, and the wild-type lysine are all common at positions equivalent to the ¹⁰Fn3 position 54. In consequence, it appears likely that the selected residues at positions 52 and 54 are at least consistent with favorable biophysical properties. In contrast, no tryptophan is found at the position equivalent to the ¹⁰Fn3 residue 53, which suggests that Tryptophan 53 may have been selected for a reason specific to the present selection, such as due to a contribution to TNF-α binding. This is consistent with the absence of this motif in later selections against other antigens, again suggesting that the PWA/G motif is more likely to contribute to TNF-α binding directly than through stability or solubility of the ¹⁰Fn3 domain. The preference for the PWA/G motif on loop DE suggests another possible reason for the preference for the BCᵣ-DEᵣ-FGᵣ library during the selection: the BCᵣ-DEᵣ-FGᵣ library alone contained the randomized DE loop, and would be expected to outcompete the other two libraries if the PWA/G sequence were important to target binding.

The sequences selected most frequently in the BC loop is NRSGLQS (12/61) (SEQ ID NO: 31), whereas the sequence selected most commonly in the ) FG loop is AQTGHHLHDK (6/61) (SEQ ID NO: 32). An NRSGLQS BC loop and an AQTGHHLHDK FG loop have not been found in the same molecule, but two clones were found which contain the most frequently isolated sequences on two of the three randomized loops. These clones, T10.06 (BC: NRSGLQS, DE: PWA) and T09.12 (DE: PWA, FG: AQTGHHLHDK), have two of the lowest four dissociation constants from TNF-α of the clones examined (Table 1).

Due to the use of a Taq polymerase that contains no proofreading activity, every round of PCR introduced additional random mutations into both the CDR-like loops and the beta-sheet scaffold of the ¹⁰Fn3 sequence, at the estimated rate of 0.01 % per base pair, i.e., 3% per ¹⁰Fn3 gene per round of PCR ) and approximately 75% per round of selection. Consequently, it is likely that the residues preserved as wild-type and those preserved in a non-wild-type stable sequence indicate that such sequences were selected due to their superior properties. In the mutated loops, it is impossible to distinguish between the mutations introduced by oligonucleotide synthesis or by PCR mutagenesis, but in the beta-strand scaffold, most of the mutations selected originate from Taq errors. The selected clones showed several conserved changes in the scaffold of the protein, which had not been randomized intentionally. Figure 18 indicates the residues in the ¹⁰Fn3 beta sheet that had not been randomized, but nevertheless mutated during selection. This mutagenesis occurred at the frequency of 26-28 of the 61 clones; these mutations are marked with arrows under the wild-type ¹⁰Fn3 sequence and with the letter that identifies the selected residue. In particular, 28 of the 61 clones mutated from Leucine 18 to Arginine or to Glutamine, and 26 clones mutated from Threonine 56 to Isoleucine. Figure 19 shows the location of these scaffold mutations. Whereas position 56 is at the stem loop DE and thus would be expected to affect the conformation and the target-binding properties of this loop, the distance of position 18 from the presumed TNF-α-binding loops suggests that the selective advantage of this mutation may arise from an indirect effect on the conformation of loop BC or from an effect on the stability of the protein (Figure 19). This is supported by an experiment in which clone T10.06, which contains the frequently seen L18R and T561 changes from the wild-type, was mutagenized to reverse position 18 back to the wild-type leucine. This change caused an increase of the K_{d} of the variant by approximately 10-fold. The weaker binding of the T10.06(L18) protein to TNF-α suggests that the residue at position 18 has an effect on the binding of the target by the CDR-like loops, possibly by a minor structural change that is transmitted through the beta-strand to loop BC.

### Affinity and Specificity of the Selected TNF-α Binding Pools

The apparent average K_{d} values of free protein pools for TNF-α after nine and after ten rounds of selection were found to be indistinguishable (4 and 6 nM, respectively; Table 1); this similarity in affinity is consistent with the relatively low (10 fold) level of enrichment observed in the last round of selection and with the similarity in the sequence composition of the two pools. The apparent average K_{d} values of free protein pool after four further rounds of selection was 3 nM, also indistinguishable from those of R9 and R10 pools

In order to assess the specificity of the binding of the pool selected after ten rounds of selection, we compared the binding of two different free protein pools to three cytokines immobilized on Sepharose to TNF-α, the target of the selection, and to IL-1α and IL-13, which were irrelevant to the selection. The first pool had been transcribed and translated from the initial, randomized DNA library before the selection (R0), and the second pool, from the library after ten rounds of selection (R10).

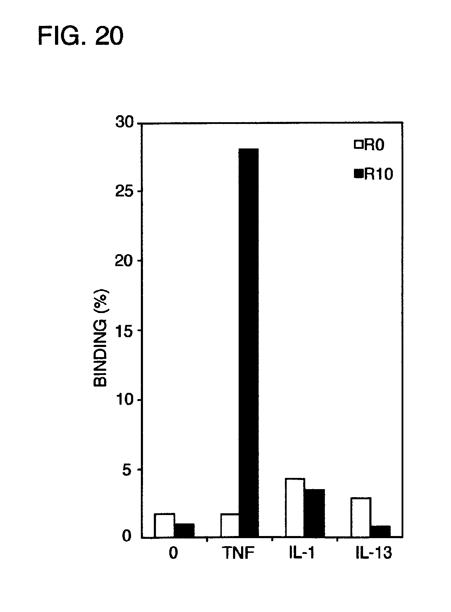

To carry out these experiments, the PCR product of the elution after the tenth round of selection was transcribed and translated *in vitro*, in the presence of ³⁵S-methionine but without forming the mRNA-protein fusion. The resulting fraction of the free protein bound to TNF-α-Sepharose, to IL-1α-Sepharose, to IL-13-Sepharose at approximately 10 µM, 30 µM, and 50 µM, respectively, and to underivatized Sepharose was compared (Figure 20), using the procedure described above for DNA/mRNA-protein fusion binding to TNF-α-Sepharose. The amount of the selected pool bound to each of the targets was measured by scintillation counting of the washed beads.

Figure 20 shows that, whereas the binding of R0 to TNF-α, IL-1α, and IL-13 was similar (2%, 4%, and 3%, respectively), the ten rounds of selection resulted in 32% binding to the targeted TNF-α, in 3% binding to IL-1α, and in 1% binding to IL-13. The absolute and the relative increase of protein binding to TNF-α demonstrates the ability of the ¹⁰Fn3 scaffold and of the DNA/mRNA-protein fusion-based selection system to select target-specific binders.

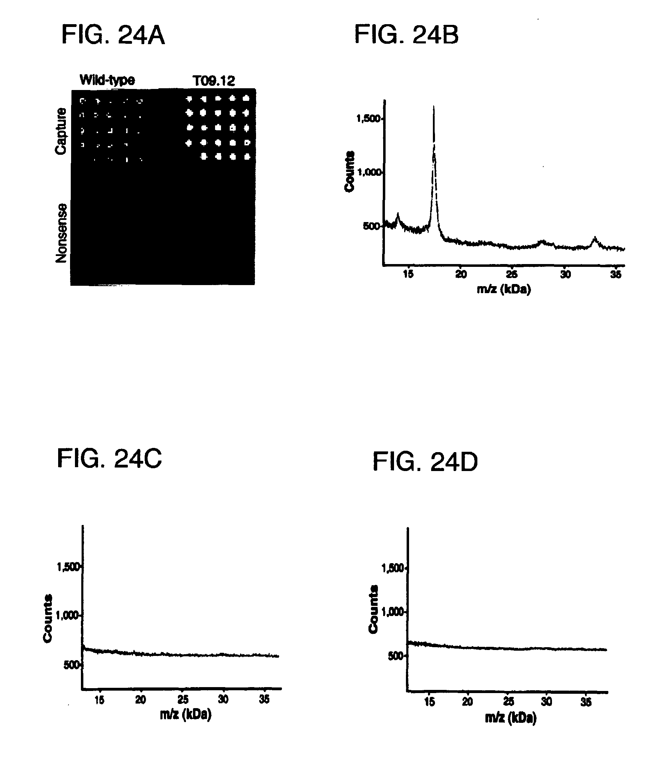

To examine the specificity of binding further, clone T09.12 was immobilized in a microarray format (as generally described below) and was tested for binding to soluble TNF-α. Specific binding of TNF-α to this clone was detected using fluorescence detection (Figure 24A) and mass spectroscopy (Figure 24B). For the mass spectroscopy results, binding assays were carried out in the presence of fetal bovine serum, an exemplary complex biological fluid containing a variety of potential interfering proteins. For fluorescence detection (Figure 24A), a mixture of RNA-¹⁰Fn3 fusion of wild-type ¹⁰Fn3 and of the T09.12 variant (Table 1) was hybridized onto a DNA microarray on which oligonucleotides complementary to the RNA portion of the fusion molecules had been immobilized at 600 micron pitch, with 24 replicate features. After removal of unhybridized fusion by washing, the surface was exposed to biotin-TNF-α (2.6 µg/mL in TBS, 0.02% Tween-20, 0.2% BSA), washed, and air-dried. The captured biotin-TNF-α was detected by Cy3-labeled anti-biotin monoclonal antibody (Sigma) using a ScanArray 5000 system (GSI Lumonics). For mass spectroscopy detection, RNA-¹⁰Fn3 fusion of the T09.12 variant (Figure 24B) and wild-type ¹⁰Fn3 (Figure 24C) was treated with RNase A to generate a fusion between the protein and the DNA linker. The resulting DNA-linked protein was hybridized to a glass coverslip arrayed with an immobilized oligonucleotide complementary to the DNA linker (Figures 24B and 24C; no fusion was applied in Figure 24D). After washing, the above surfaces were exposed to TNF-α (1.5 mg/mL in 90% v/v PBS/10% fetal bovine serum). The dried chip was spotted with MALDI matrix and analyzed with a Voyager DE MALDI-TOF mass spectrometer (PerSeptive Biosystems). A signal at 17.4 kD, which corresponded to the expected molecular mass of monomeric TNF-α, was detected on the 200 µm features that contained T09.12 protein (Figure 24A), but not on the features that contained wild-type ¹⁰Fn3 (Figure 24B) nor on the features that did not contain DNA-protein fusion (Figure 24C).

### K_{d} of the Selected TNF-α Binding Clones

Dissociation constants were determined for all the clones that were represented more than once in the two pools generated after nine and after ten rounds of selection, as well as for the only clone that originated from the BC_{wt}-DE_{wt}-FGᵣ library (clone T09.08).

To determine these binding constants, biotinylated TNF-α was prepared using the NHS-LC-LC-Biotin reagent supplied by Pierce (Rockford, IL). MALDI-TOF mass spectrometry was used to estimate that more than 80% of the monomeric TNF-α, and hence more than 99% of the trimer, was biotinylated.

For the R9 and R10 pools (and the R14 and M12 pools discussed below), as well as for the characterized clones derived from these two pools, eleven samples of 0.25 nM, *in vitro*-translated, ³⁵S-methionine-labeled free protein were incubated with the biotinylated TNF-α at a concentration between 17 pM and 23 nM, in 200 µL 10 mM HEPES, pH 7.4, 150 mM NaCl, 1% BSA, 0.02% Triton, for one hour at room temperature. Subsequently, each sample was loaded on a pre-soaked, SAM^{2R} Biotin Capture Membrane (Promega, Madison, WI) using a 96 well, Easy-Titer™ ELIFA system (Pierce). Under vacuum, each spot was washed with 200 µL of HBS pH 7.4, 1% BSA, 0.05% Triton; next the entire membrane was rinsed in the buffer and air-dried. The membrane was exposed with a Storage Phosphor Screen (Molecular Dynamics, Sunnyvale, CA) overnight, and the intensities of the resulting individual spots were quantified using a STORM 860 phosphoimager with the ImageQuaNT densitometry program (Molecular Dynamics). The K_{d} of the binding was determined by fitting the equilibrium equation to the resulting binding curve (KaleidaGraph, Synergy Software); the error of the experiment was estimated from 2-4 independent experiments.

In these studies, the K_{d} values were found to be in the narrow range of 1-24 nM (Table 1). The T09.12 and T10.06 clones, which contained the most commonly isolated sequences in two loops each, have the low K_{d} of 4 and 2 nM, respectively; on the other hand, a number of clones with less frequently seen loops, such as clones T09.07 and T10.15, showed similarly tight binding.

A sample comparison of TNF-α binding between free protein and the cDNA/mRNA-protein complex derived from the same sequence showed that the two dissociation constants were within experimental error of each other, a property of the system that makes it possible to use the cDNA/mRNA-protein complex to select for target-binding properties of the protein itself.

### High-Stringency Selection of TNF-α Binding Clones

Despite the duplicate clones isolated, the TNF-α-binding pools after nine and after ten rounds of selection contained numerous different clones, i.e., 38 different sequences in 61 clones sampled. Therefore, further selection, with more stringent binding requirements, was undertaken to recover a subset of these clones with superior TNF-α binding properties. Consequently, four further rounds of selections (R11-R14) were conducted in solution, where the concentration of the target was controlled more easily. The concentration of TNF-α was limited to 0.5 nM and the concentration of DNA/mRNA-¹⁰Fn3 pool to 0.1 nM; in addition, the length and the temperature of the washes of the ¹⁰Fn3/TNF-a complex bound to streptavidin-coated paramagnetic beads were increased.

Specifically, these selections were carried out as follows. For rounds 11-13, 0.1 nM DNA/mRNA-¹⁰Fn3 fusion library, which had been made as described above, was pre-cleared by tumbling for 1 hour at 4°C with 100 µL of Dynabeads^{®} M-280 (streptavidin-coated paramagnetic beads; Dynal, Lake Success, NY) that had been pre-blocked in Binding Buffer. The resulting pre-cleared fusion mixture was combined with 0.5 nM biotinylated TNF-α in 300 µL of the above Binding Buffer, and the complex incubated at 4°C for 1 hour. Next, 100 µl of resuspended Dynabeads^{®} M-280 Streptavidin at 1.3 g/cm³, which had been blocked by overnight incubation in Binding Buffer, were added to the mixture and incubated at 4°C, with tumbling, for 45 additional minutes. The paramagnetic beads were separated from the supernatant on a Dynal MPC-S rack, the supernatant was removed, and the beads were washed with the Binding Buffer for 1, 15, and 30 minutes in the case of R11 and R12, or for 1 minute, followed by nine ten-minute washes in the case of R13-R14. DNA was eluted from the washed DNA/mRNA-¹⁰Fn3:TNF-α-biotin:streptavidin-bead complexes with two washes of 100 µL 0.1 M KOH, and treated as described above for the column-based selection to produce the next generation DNA/mRNA-¹⁰Fn3 fusion library. Round 14 differed from R11-R13 in that the selection was performed at 30°C and in the presence of an additional 150 mM NaCl. Except for the elevated temperature, the sequence of washes was the same for R14 as for R13.

Twenty-two clones derived from the DNA eluted after four further rounds of selection (R14) were picked at random and found to represent 15 different loop sequences (Table 2; full sequences in Figure 25). The clone T10.06, isolated previously from R10 as described above, was picked eight separate times, whereas the remaining sequences, including T09.31, which had been isolated from the R9 pool, were found in one isolate each. Similar to the isolates from rounds nine and ten, the R14 clones examined showed a preference (18 of 22 clones) for the PWA/G sequence in the DE loop, and four new, non-wild-type DE sequences were revealed.

Whereas the apparent average K_{d} values of the R14 free protein pool, 3 nM, is similar to those measured for the pools after nine and ten rounds (4 and 6 nM, respectively), several K_{d} values of the clones isolated from the R14 pool were an order of magnitude lower than the lowest values observed in the R9 and R10 pools (Table 2). The clones that bound TNF-α most tightly, T14.07 and T14.25, had a K_{d} of 90 pmol. Thus, the conditions used in the last four rounds of selections were stringent enough to favor ¹⁰Fn3 molecules with subnanomolar K_{d}, but not so stringent as to eliminate such molecules.

### Mutagenic Affinity Maturation

As discussed above, the selections described herein may also be combined with mutagenesis after all or a subset of the selection steps to further increase library diversity. In one parallel selection strategy, error-prone PCR was incorporated into the amplification of DNA between rounds (Cadwell and Joyce, PCR Methods Appl 2:28 (1992)). This technique was carried out beginning with the diverse DNA pool eluted after R8 above. This pool was amplified using error-prone PCR, with the pool divided into seven equal parts and mutagenized at the target frequency of 0.8%, 1.6%, 2.4%, 3.2%, 4.0%, 4.8%, and 5.6%. The seven PCR reactions were combined, and cDNA/RNA-protein fusion was made from the mixture and subjected to a round of selection in solution. Before the second mutagenic round, M10, error-prone PCR was performed in three separate reactions, at 0.8%, 1.6%, and 2.4%. The two remaining rounds, M11 and M12, were performed using standard Taq PCR. Except for mutagenesis, the selection conditions for M9-M12 were the same as for R11-R14. The twenty M12 clones tested showed tighter binding to TNF-α than the clones selected using the two earlier selection protocols (Table 3; full sequences in Figure 25); the tightest binding of TNF-α was seen in M12.04, and had the observed K_{d} of 20 pM. These results demonstrated that low-level, random mutagenesis late in a selection can improve both the binding affinity of selected antibody mimics (20 pM vs. 90 pM) and the speed with which they can be selected (12 rounds vs. 14 rounds). In addition, the frequency of tight binders in this mutagenesis approach was observed to be about 5%, whereas the frequency is approximately 3% in other selections.

### Superiority of Fn Binders

The selection of ¹⁰Fn3 variants capable of binding to TNF-α, performed using covalent mRNA-protein fusion as the unit of selection, was won by molecules with dissociation constants as low as 20 pM. These K_{d} values compared favorably against the standards of selection of others that used other antibody mimic scaffolds and selection methods. Consequently, the ¹⁰Fn3-based scaffold and covalent mRNA-protein fusion-based *in vitro* selection method may be utilized for the development of antibody mimics against a broad range of antigens. In addition, the subnanomolar, TNF-α-binding ¹⁰Fn3 variants described herein represent potential therapeutic, research, and diagnostic agents. Moreover, since this *in vitro* selection method can be automated, such a combination of scaffold and selection methods have applications on the genomic scale.

One of the factors that contributed to the success of the present selection was the randomization of all three CDR-like loops of ¹⁰Fn3; similar libraries which contained only one or two randomized loops were less likely to include tight binders than the library with three randomized, CDR-like loops.

In the selection reported above, the randomized loops remained the length of the corresponding, wild-type ¹⁰Fn3 loops. To generate further library diversity, the length of the loops as well as their sequences may be varied, to incorporate favorable mutations in the ¹⁰Fn3 beta-sheet into the wild-type scaffold used for library construction, and to create libraries with randomized beta-sheet scaffolds which will allow selection of structures even more successful at mimicking antibodies.

Selections similar to those described herein may be carried out with any other binding species target (for example, IL-1 or IL-13).

### Animal Studies

Wild-type ¹⁰Fn3 contains an integrin-binding tripepetide motif, Arginine 78 - Glycine 79 - Aspartate 80 (the "RGD motif) at the tip of the FG loop. In order to avoid integrin binding and a potential inflammatory response based on this tripeptide in vivo, a mutant form of ¹⁰Fn3 was generated that contained an inert sequence, Serine 78 - Glycine 79 - Glutamate 80 (the "SGE mutant"), a sequence which is found in the closely related, wild-type ¹¹Fn3 domain. This SGE mutant was expressed as an N-terminally His₆-tagged, free protein in E. coli, and purified to homogeneity on a metal chelate column followed by a size exclusion column.

In particular, the DNA sequence encoding His₆-¹⁰Fn3(SGE) was cloned into the pET9a expression vector and transformed into BL21 DE3 pLysS cells. The culture was then grown in LB broth containing 50 µg/mL kanamycin at 37°C, with shaking, to A₅₆₀=1.0, and was then induced with 0.4 mM IPTG. The induced culture was further incubated, under the same conditions, overnight (14-18 hours); the bacteria were recovered by standard, low speed centrifugation. The cell pellet was resuspended in 1/50 of the original culture volume of lysis buffer (50 mM Tris 8.0, 0.5 M NaCl, 5% glycerol, 0.05% Triton X- 100, and 1 mM PMSF), and the cells were lysed by passing the resulting paste through a Microfluidics Corporation Microfluidizer M110-EH, three times. The lysate was clarified by centrifugation, and the supernatant was filtered through a 0.45 µm filter followed by filtration through a 0.2 µm filter. 100 mL of the clarified lysate was loaded onto a 5 mL Talon cobalt column (Clontech, Palo Alto, CA), washed by 70 mL of lysis buffer, and eluted with a linear gradient of 0-30 mM imidazole in lysis buffer. The flow rate through the column through all the steps was 1 mL/min. The eluted protein was concentrated 10-fold by dialysis (MW cutoff = 3,500) against 15,000-20,000 PEG. The resulting sample was dialysed into buffer 1 (lysis buffer without the glycerol), then loaded, 5 mL at a time, onto a 16 x 60 mm Sephacryl 100 size exclusion column equilibrated in buffer 1. The column was run at 0.8 mL/min, in buffer 1; all fractions that contained a protein of the expected MW were pooled, concentrated 10X as described above, then dialyzed into PBS. Endotoxin screens and animal studies were performed on the resulting sample (Toxikon; MA).

The endotoxin levels in the samples examined to date have been below the detection level of the assay. In a preliminary animal toxicology study, this protein was injected into two mice at the estimated 100X therapeutic dose of 2.6 mg/mouse. The animals survived the two weeks of the study with no apparent ill effects. These safety results support the use of ¹⁰Fn3 incorporated into an IV drug.

### Alternative Constructs for In Vivo Use

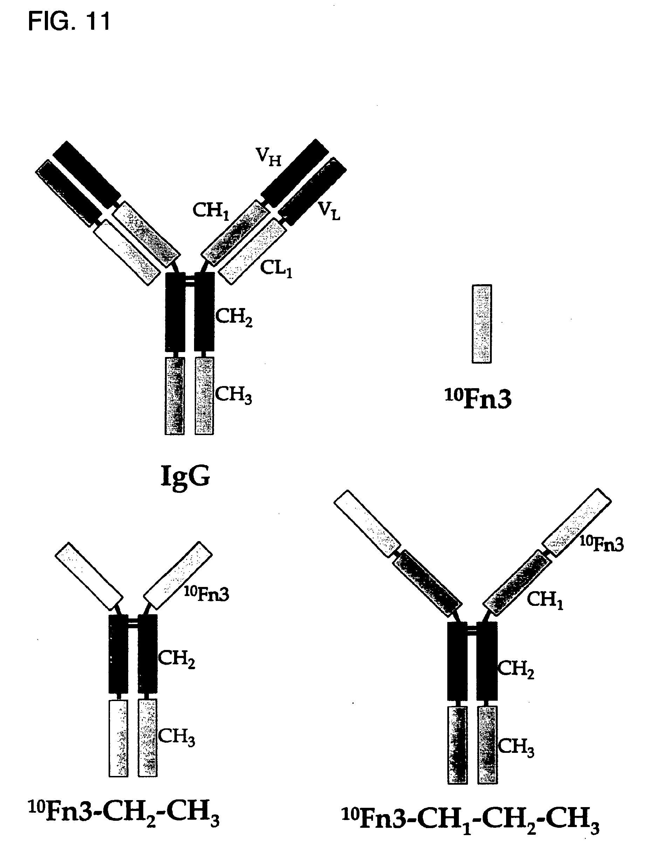

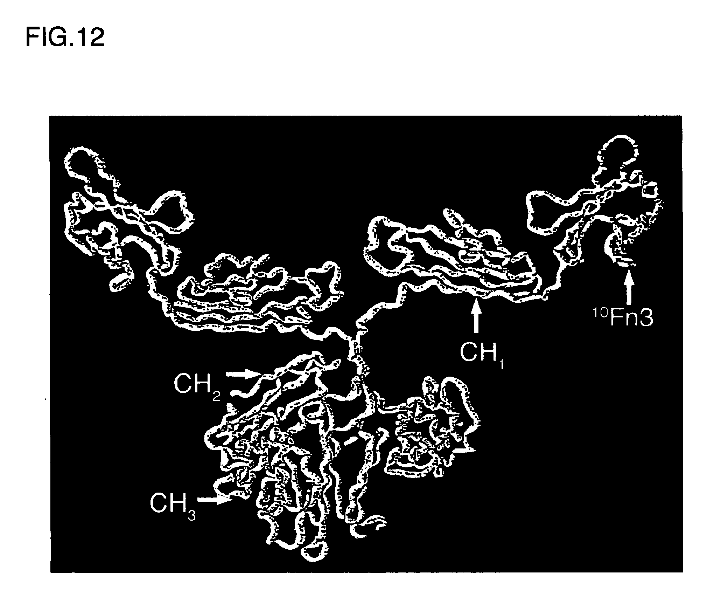

To extend the half life of the 8 kD ¹⁰Fn3 domain, a larger molecule has also been constructed that mimics natural antibodies. This ¹⁰Fn3-F_{c} molecule contains the -CH₁-CH₂-CH₃ (Figure 11) or -CH₂-CH₃ domains of the IgG constant region of the host; in these constructs, the ¹⁰Fn3 domain is grafted onto the N-terminus in place of the IgG V_{H} domain (Figures 11 and 12). Such antibody-like constructs are to improve the pharmacokinetics of the protein as well as its ability to harness the natural immune response.

In order to construct the murine form of the ¹⁰Fn3-CH₁-CH₂-CH₃ clone, the -CH₁-CH₂-CH₃ region was first amplified from a mouse liver spleen cDNA library (Clontech), then ligated into the pET25b vector. The primers used in the cloning were 5' Fc Nest and 3' 5 Fc Nest, and the primers used to graft the appropriate restriction sites onto the ends of the recovered insert were 5' Fc HIII and 3' Fc Nhe:
5' Fc Nest 5'GCG GCA GGG TTT GCT TAC TGG GGC CAA GGG 3' (SEQ ID NO: 15);
3' Fc Nest 5'GGG AGG GGT GGA GGT AGG TCA CAG TCC 3' (SEQ ID NO: 16);
3' Fc Nhe 5' TTT GCT AGC TTT ACC AGG AGA GTG GGA GGC 3' (SEQ ID NO: 17); and
5' Fc HIII 5' AAA AAG CTT GCC AAA ACG ACA CCC CCA TCT GTC 3' (SEQ ID NO: 18).

Further PCR was used to remove the CH₁ region from this clone and to create the Fc part of the shorter, ¹⁰Fn3-CH₂-CH₃ clone. The sequence encoding ¹⁰Fn3 was spliced onto the 5' end of each clone; either the wild type ¹⁰Fn3 cloned from the same mouse spleen cDNA library or a modified ¹⁰Fn3 obtained by mutagenesis or randomization of the molecules can be used. The oligonucleotides used in the cloning of murine wild-type ¹⁰Fn3 were:
Mo 5PCR-NdeI:
   5' CATATGGTTTCTGATATTCCGAGAGATCTGGAG 3' (SEQ ID NO: 19);
Mo5PCR-His-Ndel (for an alternative N-terminus with the His₆ purification tag): 
5' CAT ATG CAT CAC CAT CAC CAT CAC GTT TCT GAT ATT CCG
AGA G 3' (SEQ ID NO: 20);
 and
Mo3PCR-EcoRI: 5' GAATTCCTATGTTTTATAATTGATGGAAAC3' (SEQ ID NO: 21).

The human equivalents of the clones are constructed using the same strategy with human oligonucleotide sequences.

### Antibody Mimics in Protein Chip Applications

Any of the antibody mimics described herein may be immobilized on a solid support, such as a microchip. The suitability of the present scaffolds, for example, the ¹⁰Fn3 scaffold, for protein chip applications is the consequence of (1) their ability to support many binding functions which can be selected rapidly on the bench or in an automated setup, and (2) their superior biophysical properties.

The versatile binding properties of ¹⁰Fn3 are a function of the loops displayed by the Fn3 immunoglobulin-like, beta sandwich fold. As discussed above, these loops are similar to the complementarity determining regions of antibody variable domains and can cooperate in a way similar to those antibody loops in order to bind antigens. In our system, ¹⁰Fn3 loops BC (for example, residues 21-30), DE (for example, residues 51-56), and FG (for example, residues 76-87) are randomized either in sequence, in length, or in both sequence and length in order to generate diverse libraries of mRNA-¹⁰Fn3 fusions. The binders in such libraries are then enriched based on their affinity for an immobilized or tagged target, until a small population of high affinity binders are generated. Also, error-prone PCR and recombination can be employed to facilitate affinity maturation of selected binders. Due to the rapid and efficient selection and affinity maturation protocols, binders to a large number of targets can be selected in a short time.

As a scaffold for binders to be immobilized on protein chips, the ¹⁰Fn3 domain has the advantage over antibody fragments and single-chain antibodies of being smaller and easier to handle. For example, unlike single-chain scaffolds or isolated variable domains of antibodies, which vary widely in their stability and solubility, and which require an oxidizing environment to preserve their structurally essential disulfide bonds, ¹⁰Fn3 is extremely stable, with a melting temperature of 110°C, and solubility at a concentration > 16 mg/mL. The ¹⁰Fn3 scaffold also contains no disulfides or free cysteines; consequently, it is insensitive to the redox potential of its environment. A further advantage of ¹⁰Fn3 is that its antigen-binding loops and N-terminus are on the edge of the beta-sandwich opposite to the C-terminus; thus the attachment of a ¹⁰Fn3 scaffold to a chip by its C-terminus aligns the antigen-binding loops, allowing for their greatest accessibility to the solution being assayed. Since ¹⁰Fn3 is a single domain of only 94 amino acid residues, it is also possible to immobilize it onto a chip surface at a higher density than is used for single-chain antibodies, with their approximately 250 residues. In addition, the hydrophilicity of the ¹⁰Fn3 scaffold, which is reflected in the high solubility of this domain, minimizes unwanted binding of ¹⁰Fn3 to a chip surface.

The stability of the ¹⁰Fn3 scaffold as well as its suitability for library formation and selection of binders are likely to be shared by the large, Fn3-like class of protein domains with an immunoglobulin-like fold, such as the domains of tenascin, N-cadherin, E-cadherin, ICAM, titin, GCSF-R, cytokine receptor, glycosidase inhibitor, and antibiotic chromoprotein. The key features shared by all such domains are a stable framework provided by two beta-sheets, which are packed against each other and which are connected by at least three solvent-accessible loops per edge of the sheet; such loops can be randomized to generate a library of potential binders without disrupting the structure of the framework (as described above). In addition, as with ¹⁰Fn3, any of these loops (or similar loops from other proteins) may be immobilized alone or in combination with other loops onto a solid support surface.

### Immobilization of Fn3-Based Antibody Mimics

To immobilize antibody mimics, such as Fn3-based antibody mimics, to a chip surface, a number of exemplary techniques may be utilized. For example, such antibody mimics may be immobilized as RNA-protein fusions by Watson-Crick hybridization of the RNA moiety of the fusion to a base complementary DNA immobilized on the chip surface (as described, for example, in Addressable Protein Array, U.S.S.N. 60/080,686; U.S.S.N. 09/282,734; and WO 99/51773; and Methods for Encoding and Sorting In Vitro Translated Proteins, U.S.S.N. 60/151,261 and U.S.S.N. 09/648,040). Alternatively, antibody mimics can be immobilized as free proteins directly on a chip surface. Manual as well as robotic devices may be used for deposition of the antibody mimics on the chip surface. Spotting robots can be used for deposition of antibody mimics with high density in an array format (for example, by the method of Lurking et al., Anal Biochem. 1999 May 15;270(1):103-11). Different methods may also be utilized for anchoring the antibody mimic on the chip surface. A number of standard immobilization procedures may be used including those described in Methods in Enzymology (K. Mosbach and B. Danielsson, eds.), vols. 135 and 136, Academic Press, Orlando, Florida, 1987; Nilsson et al., Protein Expr. Purif. 1997 Oct;11(1):1-16; and references therein. Oriented immobilization of antibody mimics can help to increase the binding capacity of chip-bound antibody mimics. Exemplary approaches for achieving oriented coupling are described in Lu et al., The Analyst (1996), vol. 121, p. 29R-32R; and Turkova, J Chromatogr B Biomed Sci App. 1999 Feb 5;722(1-2):11-31. In addition, any of the methods described herein for anchoring antibody mimics to chip surfaces can also be applied to the immobilization of antibody mimics on beads, or other supports.

### Target Protein Capture and Detection

Selected populations of scaffold-binders may be used for detection and/or quantitation of analyte targets, for example, in samples such as biological samples. To carry out this type of diagnostic assay, selected scaffold-binders to targets of interest are immobilized on an appropriate support to form multi-featured protein chips. Next, a sample is applied to the chip, and the components of the sample that associate with the binders are identified based on the target-specificity of the immobilized binders. Using this technique, one or more components may be simultaneously identified or quantitated in a sample (for example, as a means to carry out sample profiling).

Methods for target detection allow measuring the levels of bound protein targets and include, without limitation, radiography, fluorescence scanning, mass spectroscopy (MS), and surface plasmon resonance (SPR). Autoradiography using a phosphorimager system (Molecular Dynamics, Sunnyvale, CA) can be used for detection and quantification of target protein which has been radioactively labeled, e.g., using ³⁵S methionine. Fluorescence scanning using a laser scanner (see below) may be used for detection and quantification of fluorescently labeled targets. Alternatively, fluorescence scanning may be used for the detection of fluorescently labeled ligands which themselves bind to the target protein (e.g., fluorescently labeled target-specific antibodies or fluorescently labeled streptavidin binding to target-biotin, as described below).

Mass spectroscopy can be used to detect and identify bound targets based on their molecular mass. Desorption of bound target protein can be achieved with laser assistance directly from the chip surface as described below. Mass detection also allows determinations, based on molecular mass, of target modifications including post-translational modifications like phosophorylation or glycosylation. Surface plasmon resonance can be used for quantification of bound protein targets where the scaffold-binder(s) are immobilized on a suitable gold-surface (for example, as obtained from Biacore, Sweden).

Described below are exemplary schemes for selecting binders (in this case, Fn-binders specific for the protein, TNF-α) and the use of those selected populations for detection on chips. This example is provided for the purpose of illustrating the invention, and should not be construed as limiting.

### Selection of TNF-α Binders Based on ¹⁰Fn3 Scaffold

In one exemplary use for scaffold selection on chips, an ¹⁰Fn3-based selection was performed against TNF-α, using a library of human ¹⁰Fn3 variants with randomized loops BC, DE, and FG. The library was constructed from three DNA fragments, each of which contained nucleotide sequences that encoded approximately one third of human ¹⁰Fn3, including one of the randomized loops. The DNA sequences that encoded the loop residues listed above were rebuilt by oligonucleotide synthesis, so that the codons for the residues of interest were replaced by (NNS)ₙ, where N represents any of the four deoxyribonucleotides (A, C, G, or T), and S represents either C or G. The C-terminus of each fragment contained the sequence for the FLAG purification tag.

Once extended by Klenow, each DNA fragment was transcribed, ligated to a puromycin-containing DNA linker, and translated in vitro, as described by Szostak et al. (Roberts and Szostak, Proc. Natl. Acad. Sci USA 94:12297, 1997; Szostak et al., U.S.S.N. 09/007,005 and U.S.S.N. 09/247,190; Szostak et al., WO98/31700), to generate an mRNA-peptide fusion, which was then reverse-transcribed into a DNA-mRNA-peptide fusion. The binding of the FLAG-tagged peptide to M2 agarose separated full-length fusion molecules from those containing frameshifts or superfluous stop codons; the DNA associated with the purified full-length fusion was amplified by PCR, then the three DNA fragments were cut by Ear I restriction endonuclease and ligated to form the full length template. The template was transcribed, ligated to puromycin-containing DNA linkers, and translated to generate a ¹⁰Fn3-RNA/cDNA library, which was then reverse-transcribed to yield the DNA-mRNA-peptide fusion library which was subsequently used in the selection.

Selection for TNF-α binders took place in 50 mM HEPES, pH 7.4, 0.02% Triton-X, 0.1 mg/mL salmon sperm DNA. The PROfusion™ library was incubated with Sepharose-immobilized TNF-α; after washing, the DNA associated with the tightest binders was eluted with 0.1 M KOH, amplified by PCR, and transcribed, ligated, translated, and reverse-transcribed into the starting material for the next round of selection.

Ten rounds of such selection were performed (as shown in Figure 13); they resulted in a PROfusion™ pool that bound to TNF-α-Sepharose with the apparent average K_{d} of 120 nM. Specific clonal components of the pool that were characterized showed TNF-α binding in the range of 50-500 nM.

### Immobilization, Target Protein Capture, and MALDI-TOF Detection

As a first step toward immobilizing Fn3 fusions to a chip surface, an oligonucleotide capture probe was prepared with an automated DNA synthesizer (PE BioSystems Expedite 8909) using the solid-support phosphoramidite approach. All reagents were obtained from Glen Research. Synthesis was initiated with a solid support containing a disulfide bond to eventually provide a 3'-terminal thiol functionality. The first four monomers to be added were hexaethylene oxide units, followed by 20 T monomers. The 5'-terminal DMT group was not removed. The capture probe was cleaved from the solid support and deprotected with ammonium hydroxide, concentrated to dryness in a vacuum centrifuge, and purified by reverse-phase HPLC using an acetonitrile gradient in triethylammonium acetate buffer. Appropriate fractions from the HPLC were collected, evaporated to dryness in a vacuum centrifuge, and the 5'-terminal DMT group was removed by treatment with 80% AcOH for 30 minutes. The acid was removed by evaporation, and the oligonucleotide was then treated with 100 mM DTT for 30 minutes to cleave the disulfide bond. DTT was removed by repeated extraction with EtOAc. The oligonucleotide was ethanol precipitated from the remaining aqueous layer and checked for purity by reverse-phase HPLC.

The 3'-thiol capture probe was adjusted to 250 µM in degassed 1X PBS buffer and applied as a single droplet (75 µL) to a 9x9mm gold-coated chip (Biacore) in an argon-flushed chamber containing a small amount of water. After 18 hours at room temperature, the capture probe solution was removed, and the functionalized chip was washed with 50 mL 1X PBS buffer (2x for 15 minutes each) with gentle agitation, and then rinsed with 50 mL water (2x for 15 minutes each) in the same fashion. Remaining liquid was carefully removed and the functionalized chips were either used immediately or stored at 4°C under argon.

About 1pmol of ¹⁰Fn3 fusion pool from the Round 10 TNF-α selection (above) was treated with RNAse A for several hours, adjusted to 5X SSC in 70 µL, and applied to a functionalized gold chip from above as a single droplet. A 50 µL volume gasket device was used to seal the fusion mixture with the functionalized chip, and the apparatus was continuously rotated at 4°C. After 18 hours the apparatus was disassembled, and the gold chip was washed with 50 mL 5X SSC for 10 minutes with gentle agitation. Excess liquid was carefully removed from the chip surface, and the chip was passivated with a blocking solution (1X TBS + 0.02% Tween-20 + 0.25% BSA) for 10 minutes at 4°C. Excess liquid was carefully removed, and a solution containing 500 µg/mL TNF-α in the same composition blocking solution was applied to the chip as a single droplet and incubated at 4°C for two hours with occasional mixing of the droplet via Pipetman. After removal of the binding solution, the chip was washed for 5 minutes at 4°C with gentle agitation (50 mL 1X TBS + 0.02% Tween-20) and then dried at room temperature. A second chip was prepared exactly as described above, except fusion was not added to the hybridization mix.

Next, MALDI-TOF matrix (15 mg/mL 3,5-dimethoxy-4-hydroxycinnamic acid in 1:1 ethanol/10% formic acid in water) was uniformly applied to the gold chips with a high-precision 3-axis robot (MicroGrid, BioRobotics). A 16-pin tool was used to transfer the matrix from a 384-well microtiter plate to the chips, producing 200 micron diameter features with a 600 micron pitch. The MALDI-TOF mass spectrometer (Voyager DE, PerSeptive Biosystems) instrument settings were as follows: Accelerating Voltage = 25k, Grid Voltage = 92%, Guide Wire Voltage = 0.05%, Delay = 200 on, Laser Power = 2400, Low Mass Gate = 1500, Negative Ions - off. The gold chips were individually placed on a MALDI sample stage modified to keep the level of the chip the same as the level of the stage, thus allowing proper flight distance. The instrument's video monitor and motion control system were used to direct the laser beam to individual matrix features.

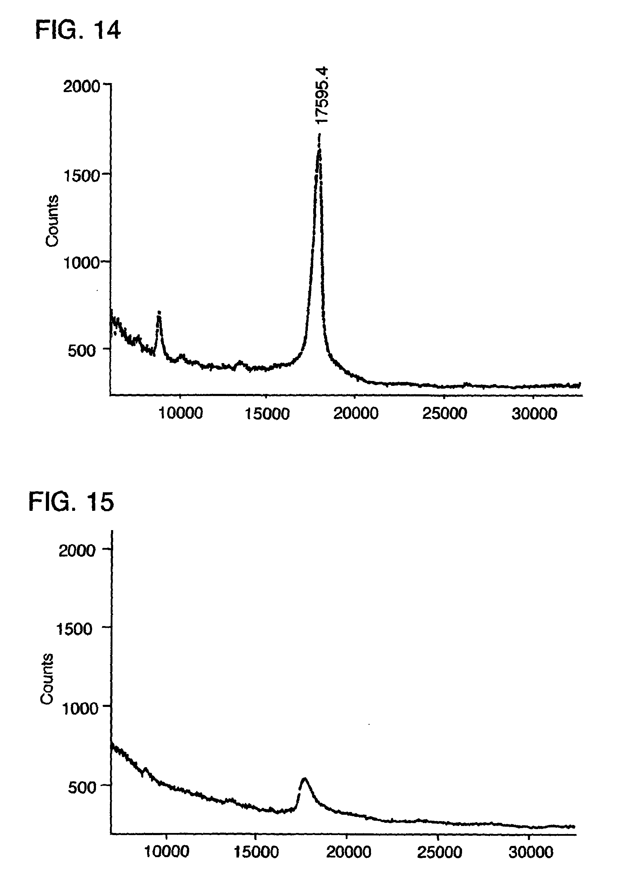

Figures 14 and 15 show the mass spectra from the ¹⁰Fn3 fusion chip and the non-fusion chip, respectively. In each case, a small number of 200 micron features were analyzed to collect the spectra, but Figure 15 required significantly more acquisitions. The signal at 17.4 kDa corresponds to TNF-α monomer.

### Immobilization Target Protein Capture, and Fluorescence Detection

Pre-cleaned 1x3 inch glass microscope slides (Goldseal, #3010) were treated with Nanostrip (Cyantek) for 15 minutes, 10% aqueous NaOH at 70°C for 3 minutes, and 1% aqueous HCl for 1 minute, thoroughly rinsing with deionized water after each reagent. The slides were then dried in a vacuum desiccator over anhydrous calcium sulfate for several hours. A 1% solution of aminopropytrimethoxysilane in 95% acetone / 5% water was prepared and allowed to hydrolyze for 20 minutes. The glass slides were immersed in the hydrolyzed silane solution for 5 minutes with gentle agitation. Excess silane was removed by subjecting the slides to ten 5-minute washes, using fresh portions of 95% acetone / 5% water for each wash, with gentle agitation. The slides were then cured by heating at 110°C for 20 minutes. The silane treated slides were immersed in a freshly prepared 0.2% solution of phenylene 1,4-diisothiocyanate in 90% DMF / 10% pyridine for two hours, with gentle agitation. The slides were washed sequentially with 90% DMF / 10% pyridine, methanol, and acetone. After air drying, the functionalized slides were stored at 0°C in a vacuum desiccator over anhydrous calcium sulfate. Similar results were obtained with commercial amine-reactive slides (3-D Link, Surmodics).

Oligonucleotide capture probes were prepared with an automated DNA synthesizer (PE BioSystems Expedite 8909) using conventional phosphoramidite chemistry. All reagents were from Glen Research. Synthesis was initiated with a solid support bearing an orthogonally protected amino functionality, whereby the 3'-terminal amine is not unmasked until final deprotection step. The first four monomers to be added were hexaethylene oxide units, followed by the standard A, G, C and T monomers. All capture oligo sequences were cleaved from the solid support and deprotected with ammonium hydroxide, concentrated to dryness, precipitated in ethanol, and purified by reverse-phase HPLC using an acetonitrile gradient in triethylammonium acetate buffer. Appropriate fractions from the HPLC were collected, evaporated to dryness in a vacuum centrifuge, and then coevaporated with a portion of water.

The purified, amine-labeled capture oligos were adjusted to a concentration of 250 µM in 50 mM sodium carbonate buffer (pH 9.0) containing 10% glycerol. The probes were spotted onto the amine-reactive glass surface at defined positions in a 5x5x6 array pattern with a 3-axis robot (MicroGrid, BioRobotics). A 16-pin tool was used to transfer the liquid from 384-well microtiter plates, producing 200 micron features with a 600 micron pitch. Each sub-grid of 24 features represents a single capture probe (i.e., 24 duplicate spots). The arrays were incubated at room temperature in a moisture-saturated environment for 12-18 hours. The attachment reaction was terminated by immersing the chips in 2% aqueous ammonium hydroxide for five minutes with gentle agitation, followed by rinsing with distilled water (3X for 5 minutes each). The array was finally soaked in 10X PBS solution for 30 minutes at room temperature, and then rinsed again for 5 minutes in distilled water.

Specific and thermodynamically isoenergetic sequences along the ¹⁰Fn3 mRNA were identified to serve as capture points to self-assemble and anchor the ¹⁰Fn3 protein. The software program HybSimulator v4.0 (Advanced Gene Computing Technology, Inc.) facilitated the identification and analysis of potential capture probes. Six unique capture probes were chosen and printed onto the chip, three of which are complementary to common regions of the ¹⁰Fn3 fusion pool's mRNA (CP3', CP5', and CPflag). The remaining three sequences (CPneg1, CPneg2, and CPneg3) are not complementary and function in part as negative controls. Each of the capture probes possesses a 3'-amino terminus and four hexaethylene oxide spacer units, as described above. The following is a list of the capture probe sequences that were employed (5'-3'):

| | |
|---|---|
| CP3': | TGTAAATAGTAATTGTCCC (SEQ ID NO: 22) |
| CP5': | TTTTTTTTTTTTTTTTTTTT(SEQ ID NO: 23) |
| CPneg1: | CCTGTAGGTGTCCAT (SEQ ID NO: 24) |
| CPflag: | CATCGTCCTTGTAGTC (SEQ ID NO: 25) |
| CPneg2: | CGTCGTAGGGGTA (SEQ ID NO: 26) |
| CPneg3: | CAGGTCTTCTTCAGAGA (SEQ ID NO: 27) |

About 1pmol of ¹⁰Fn3 fusion pool from the Round 10 TNF-α selection was adjusted to 5X SSC containing 0.02% Tween-20 and 2 mM vanadyl ribonucleotide complex in a total volume of 350 µL. The entire volume was applied to the microarray under a 400 µL gasket device and the assembly was continuously rotated for 18 hours at room temperature. After hybridization the slide was washed sequentially with stirred 500 mL portions of 5X SSC, 2.5X SSC, and 1X SSC for 5 minutes each. Traces of liquid were removed by centrifugation and the slide was allowed to air-dry.

Recombinant human TNF-α (500 µg, lyophilized, from PreproTech) was taken up in 230 µL 1X PBS and dialyzed against 700 mL stirred 1X PBS at 4°C for 18 hours in a Microdialyzer unit (3,500 MWCO, Pierce). The dialyzed TNF-α was treated with EZ-Link NHS-LC-LC biotinylation reagent (20 µg, Pierce) for 2 hours at 0°C, and again dialyzed against 700 mL stirred 1X PBS at 4°C for 18 hours in a Microdialyzer unit (3,500 MWCO, Pierce). The resulting conjugate was analyzed by MALDI-TOF mass spectrometry and was found to be almost completely functionalized with a single biotin moiety.

Each of the following processes was conducted at 4°C with continuous rotation or mixing. The protein microarray surface was passivated by treatment with 1X TBS containing 0.02% Tween-20 and 0.2% BSA (200 µL) for 60 minutes. Biotinylated TNF-α (100 nM concentration made up in the passivation buffer) was contacted with the microarray for 120 minutes. The microarray was washed with 1X TBS containing 0.02% Tween-20 (3X 50 mL, 5 minutes each wash). Fluorescently labeled streptavidin (2.5 µg/mL Alexa 546-streptavidin conjugate from Molecular Probes, made up in the passivation buffer) was contacted with the microarray for 60 minutes. The microarray was washed with 1X TBS containing 0.02% Tween-20 (2X 50 mL, 5 minutes each wash) followed by a 3 minute rinse with 1X TBS. Traces of liquid were removed by centrifugation, and the slide was allowed to air-dry at room temperature.

Fluorescence laser scanning was performed with a GSI Lumonics ScanArray 5000 system using 10 µM pixel resolution and preset excitation and emission wavelengths for Alexa 546 dye. Phosphorimage analysis was performed with a Molecular Dynamics Storm system. Exposure time was 48 hours with direct contact between the microarray and the phosphor storage screen. Phosphorimage scanning was performed at the 50 micron resolution setting, and data was extracted with ImageQuant v.4.3 software.

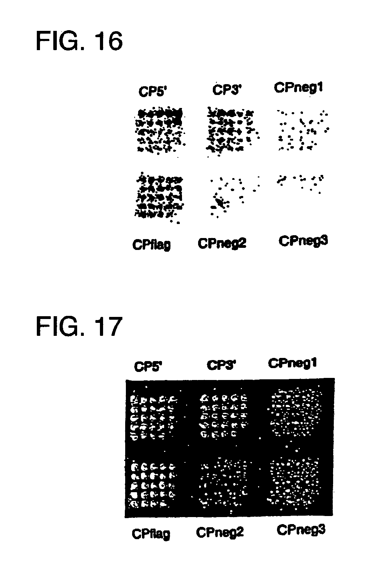

Figures 16 and 17 are the phosphorimage and fluorescence scan, respectively, of the same array. The phosphorimage shows where the ¹⁰Fn3 fusion hybridized based on the ³⁵S methionine signal. The fluorescence scan shows where the labeled TNF-α bound.

### Other Embodiments

Other embodiments are within the claims.

All publications, patents, and patent applications mentioned herein are hereby incorporated by reference.

## Claims

1. A protein comprising a tenth fibronectin type III domain (¹⁰Fn3) wherein the amino acid sequence of at least one loop selected from the group consisting of the BC loop, the DE loop and the FG loop comprises one or more amino acid alterations relative to the sequence of a naturally occurring human tenth fibronectin type III domain, said protein being **characterized by** its ability to bind to a compound with a Kd at least as tight as 10 nM.

2. The protein of claim 1, wherein the protein binds to said compound with a Kd at least as tight as 1 nM, 500 pM, 100 pM, or 20 pM.

3. The protein of claim 1 or 2, wherein the amino acid sequence of the naturally occurring human ¹⁰Fn3 is set forth in figure 4.

4. The protein of claim 3, wherein the BC loop of the naturally occurring human ¹⁰Fn3 runs between amino acid residues 21-31 of the sequence as set forth in figure 4; the DE loop of the naturally occurring human ¹⁰Fn3 runs between amino acid residues 51-56 of the sequence as set forth in figure 4; and the FG loop of the naturally occurring human ¹⁰Fn3 runs between amino acid residues 76-88 of the sequence as set forth in figure 4.

5. The protein of any one of claims 1 to 4, wherein the amino acid sequence of each of the BC loop, the DE loop and the FG loop comprises one or more amino acid alterations relative to the sequence of the naturally occurring human tenth fibronectin type III domain.

6. The protein of any one of claims 1 to 5, wherein said ¹⁰Fn3 lacks an integrin-binding motif.

7. The protein of any one of claims 1 to 6, wherein said protein is covalently bound to a nucleic acid and said nucleic acid encodes said protein.

8. The protein of claim 7, wherein said nucleic acid is RNA.

9. The protein of claim 1, wherein the compound is tumor necrosis factor-a (TNF-α).

10. The protein of claim 1, wherein said protein comprises any one of the sequences of Figure 25 (SEQ ID NOs: 34-140).

11. A fusion protein comprising the protein of any one of claims 1 to 10.

12. A composition comprising the protein of any one of claims 1 to 10 and a physiologically acceptable carrier.

13. A protein of any one of claims 1 to 10 for use in therapy or diagnosis.

14. A nucleic acid encoding the protein of any one of claims 1 to 10.

15. A method for obtaining a protein of any one of claims 1 to 10, said method comprising:
(a) providing a tenth fibronectin type III domain;
(b) generating mutated derivatives of said fibronectin type III domain by randomizing at least one loop selected from the group consisting of the BC loop, the DE loop and the FG loop of a tenth fibronectin type III domain (¹⁰Fn3), thereby producing a library of proteins comprising a tenth fibronectin type III domain wherein the amino acid sequence of the loop comprises one or more amino acid alterations relative to the sequence of a naturally occurring human tenth fibronectin type III domain;
(c) contacting said library with the compound;
(d) selecting from said library at least one protein which binds to said compound with a Kd at least as tight as 1 µM,
(e) repeating steps (b)-(d) substituting for the fibronectin type III domain in repeated step (b) the at least one protein from the previous step (d) and selecting at least one protein which binds to said compound with a Kd at least as tight as 10 nM, whereby a protein that binds with a Kd at least as tight as 10 nM to the compound is obtained.
